# EUROPEAN PATENT SPECIFICATION

(11) **EP 2 294 918 B1**
(45) Date of publication and mention of the grant of the patent: **10.10.2012**
(21) Application number: 10183935.5
(22) Date of filing: 29.08.2000
(51) Int. Cl.: C12N 9/22, A61K 38/46

(54) **Use of a ribonuclease of the T2 family having actin-binding activity for inhibiting tumor angiogenesis**
Gebrauch einer Ribonuclease der T2-Familie mit Actin-bindender Aktivität zur Hemmung der Tumorangiogenese
Utilisation d'une ribonucléase de la famille t2 ayant une activité de fixation à l'actine pour inhiber l'angiogenèse de tumeurs

(30) Priority: 30.08.1999 US 385411
(43) Date of publication of application: 16.03.2011
(62) Divisional of application: 08168000.1
(73) Proprietor: Yissum Research Development Company of the Hebrew University of Jerusalem Ltd., 91042 Jerusalem (IL)
(72) Inventor: Roiz, Levava, 55654, Kiryat-Ono (IL); Shoseyov, Oded, 99797, Karmei Yoseef (IL); Schwartz, Betty, 76667, Rechovot (IL); Smirnoff, Patricia, 76823, Rehovot (IL)
(74) Representative: Fichter, Robert Arno

(56) References cited:
- EP-B1- 1 207 755
- WO-A2-2006/035439
- US-A- 5 786 457
- KAWATA-Y ET AL.: "AMINO-ACID SEQUENCE OF RIBONUCLEASE T2 FROM ASPERGILLUS ORYZAE", EUROPEAN JOURNAL OF BIOCHEMISTRY, vol. 176, 1988, pages 683-697, XP002935748, Berlin, De
- KAWATA Y ET AL: "IDENTIFICATION OF TWO ESSENTIAL HISTIDINE RESIDUES OF RNASE T-2 FROM ASPERGILLUS-ORYZAE", EUROPEAN JOURNAL OF BIOCHEMISTRY, vol. 187, no. 1, 1990, pages 255-262, XP008009203, ISSN: 0014-2956
- NOMACHI Y ET AL: "PURIFICATION AND SOME PROPERTIES OF 2 ACID RNASE FRACTIONS EC-3.1.27.1 EC-3.1.27.3 FROM THE MYCELIA OF ASPERGILLUS-NIGER", JOURNAL OF GENERAL AND APPLIED MICROBIOLOGY, vol. 26, no. 6, 1980, pages 375-386, XP008009205, ISSN: 0022-1260
- LACCETTI P ET AL: "SEMINAL RIBONUCLEASE INHIBITS TUMOR GROWTH AND REDUCES THE METASTATIC POTENTIAL OF LEWIS LUNG CARCINOMA", CANCER RESEARCH, AMERICAN ASSOCIATION FOR CANCER RESEARCH, BALTIMORE, MD, US, vol. 54, 15 August 1994 (1994-08-15), pages 4253-4256, XP002000755, ISSN: 0008-5472
- IRIE M: "STRUCTURE-FUNCTION RELATIONSHIPS OF ACID RIBONUCLEASES: LYSOSOMAL, VACUOLAR, AND PERIPLASMIC ENZYMES", PHARMACOLOGY AND THERAPEUTICS, ELSEVIER, GB, vol. 81, no. 2, 1 January 1999 (1999-01-01), pages 77-89, XP002935746, ISSN: 0163-7258
- MASTRONICOLA M R ET AL: "Key extracellular and intracellular steps in the antitumor action of seminal ribonuclease.", EUROPEAN JOURNAL OF BIOCHEMISTRY / FEBS 15 MAY 1995, vol. 230, no. 1, 15 May 1995 (1995-05-15), pages 242-249, XP002565611, ISSN: 0014-2956
- TRUBIA M ET AL: "Mammalian Rh/T2/S-Glycoprotein Ribonuclease Family Genes: Cloning of a Human Member Located in a Region of Chromosome 6 (6q27) Frequently Deleted in Human Malignancies", GENOMICS, ACADEMIC PRESS, SAN DIEGO, US, vol. 42, no. 2, 1 June 1997 (1997-06-01), pages 342-344, XP004459303, ISSN: 0888-7543
- FETT J W ET AL: "Isolation and characterization of angiogenin, an angiogenic protein from human carcinoma cells.", BIOCHEMISTRY 24 SEP 1985 LNKD- PUBMED:4074709, vol. 24, no. 20, 24 September 1985 (1985-09-24), pages 5480-5486, XP002618087, ISSN: 0006-2960
- ACQUATI FRANCESCO ET AL: "Tumor and metastasis suppression by the human RNASET2 gene.", INTERNATIONAL JOURNAL OF ONCOLOGY MAY 2005, vol. 26, no. 5, May 2005 (2005-05), pages 1159-1168, XP009068627, ISSN: 1019-6439
- LELAND P A ET AL: "Cancer chemotherapy--ribonucleases to the rescue.", CHEMISTRY & BIOLOGY MAY 2001, vol. 8, no. 5, May 2001 (2001-05), pages 405-413, XP002565612, ISSN: 1074-5521
- DESHPANDE R A ET AL: "RIBONUCLEASES FROM T2 FAMILY", CRITICAL REVIEWS IN MICROBIOLOGY, CRC PRESS, INC., BOCA RATON, FL, US, vol. 28, no. 2, 1 June 2002 (2002-06-01), pages 79-122, XP008009078,
- LUHTALA NATALIE ET AL: "T2 Family ribonucleases: ancient enzymes with diverse roles.", TRENDS IN BIOCHEMICAL SCIENCES MAY 2010 LNKD- PUBMED:20189811, vol. 35, no. 5, May 2010 (2010-05), pages 253-259, XP002618088, ISSN: 0968-0004
- ROIZ LEVAVA ET AL: "ACTIBIND, an actin-binding fungal T2-RNase with antiangiogenic and anticarcinogenic characteristics.", CANCER 15 MAY 2006 LNKD- PUBMED:16586499, vol. 106, no. 10, 15 May 2006 (2006-05-15) , pages 2295-2308, XP002618089, ISSN: 0008-543X

## Description

### FIELD AND BACKGROUND OF THE INVENTION

The present invention relates to the use of a ribonuclease of the T2 family or a polynucleotide encoding same for , inhibiting tumor angiogenesis in a subject. The present disclosure further relates to pharmaceutical compositions containing, as an active ingredient, a ribonuclease of the T2 family or a polynucleotide encoding same for treating proliferative diseases or disorders in general and cancer in particular.

There is an ongoing interest, both within the medical community and among the general population, in the development of novel therapeutic agents for the treatment of cell proliferative diseases and disorders such as cancer.

Agents that display anti-proliferative, anti-colonization, anti-differentiation and/or anti-development properties against mammalian cells can potentially be used as anti-cancer drugs. As such, these agents are widely sought for from both natural as well as synthetic sources.

RIBASES are ribonucleases (RNases) which display a biological activity which is distinct from their ability to degrade RNA. RIBASES and their structural homologous are known to effect a large number of cellular reactions (Rybak, M. et al., . 1991, J. Biol. Chem. 266:21202-21207; Schein, C.H. 1997 Nature Biotechnol. 15:529-536). EDN and ECP, two major proteins found in the secretory granules of cytotoxic eosinophyles (members of RNase A family) are thought to participate in the immune response. In self-incompatible plants stylar S-RNases (members of RNase T2 family), arrest pollen tube growth and thus prevent fertilization. RC-RNase, produced from Bullfrog oocytes, inhibits, *in vitro,* the growth of tumor cells such as the P388, and L1210 leukemia cell lines and is effective for *in vivo* killing of sarcoma 180, Erlich, and Mep II ascites cells (Chang, C-F. et al 1988, J. Mol Biol 283:231-244). Some RNases display limited ribonuclease activity, an example of which includes angiogenins that stimulate blood vessels formation (Fett, J.W. 1985, Biochemistry 24:5480-5486).

Living organisms use extracellular RNases for defense against pathogens and tumor cells. For example, ECP is secreted in response to parasite attack (Newton, DL. 1992, J. Biol. Chem. 267:19572-19578) and displays antibacterial and antiviral activity. This activity is also displayed by Zinc-α₂-glycoprotein (Znα₂gp), an RNase present in most human body fluids including blood, seminal plasma, breast milk, synovial fluid, saliva, urine and sweat (Lei G, et al., 1998, Arch Biochem Biophys. Jul 15;355(2):160-4).

The specific mechanism by which extracellular RNases function in cellular reactions is unknown.

The main barrier to the cytotoxic activity of some RNase is the cell membrane. ECP was found to form channels in both artificial and cellular membranes. Presumably, ECP released from the granule membrane along with EDN (eosinophylic RNase, which is responsible for cerebellar Purkinjie cell destruction) transfers EDN into the intercellular space. The entrance of the fungal toxin α-sarcin (a member of the RNase A family) into target cells depends upon viral infection which permeabilizes the cellular membrane (Rybak, M. et al., 1991, J. Biol. Chem. 266:21202-21207). It is also possible that RNases enter the cell via endocytosis. When the Golgi-disrupting drugs retinoic acid or monensin were used to artificially deliver BS-RNase into the cells, cytotoxicity increased dramatically (Wu Y, et al., 1995, J Biol Chem. 21;270(29):17476-81).

Cytotoxicity of RNases can be used for therapeutic purposes. Human RNase L is activated by interferon and inhibits viral growth. Expression of the gene for human RNase L together with that for a 2'5'-A synthetase in tobacco plants is sufficient to protect plants from cucumber mosaic virus and to prevent replication of potato virus Y. Human immunodeficiency virus-1 (HIV-1) induces blockade in the RNase L antiviral pathways (Schein, C.H. 1997 Nature Biotechnol. 15:529-536.). RNases can be fused with specific membranal protein antibodies to create immunotoxins. For example, fusion of RNase A with antibodies to the transferrin receptor or to the T cell antigen CD5 lead to inhibition of protein synthesis in tumor cells carrying a specific receptor for each of the above toxins (Rybak, M. et al., 1991, J. Biol. Chem. 266:21202-21207; Newton DL, et al., 1998, Biochemistry 14;37(15):5173-83). Since RNases are less toxic to animals, they may have fewer undesirable side effect than the currently used immunotoxins.

The cytotoxicity of cytotoxic ribonucleases appears to be inversely related to the strength of the interaction between a ribonuclease inhibitor (RI) and the RNase. Ribonuclease inhibitor (RI) is a naturally occurring molecule found within vertebrate cells which serves to protect these cells from the potentially lethal effects of ribonucleases. The ribonuclease inhibitor is a 50 kDa cytosolic protein that binds to RNases with varying affinity. For example, RI binds to members of the bovine pancreatic ribonuclease A (RNase A) superfamily of ribonucleases with inhibition constants that span ten orders of magnitude, with Ki's ranging from 10⁻⁶ to 10⁻¹⁶ M.

### A-RNases

ONCONASE, like RNase A and BS-RNase, is a member of the RNase A superfamily. Members of the RNase A superfamily share about 30 % identity in amino acid sequences. The majority of non-conserved residues are located in surface loops, and appear to play a significant role in the dedicated biological activity of each RNase. ONCONASE was isolated from Northern Leopard frog (*Rana pipiens*) oocytes and early embryos. It has anti-tumor effect on a variety of solid tumors, both *in situ* and *in vivo* (Mikulski S.M., et al., 1990 J. Natl. Cancer 17;82(2):151-3). ONCONASE has also been found to specifically inhibit HIV-1 replication in infected H9 leukemia cells at non-cytotoxic concentration (Youle R.J., et al., 1994, Proc. Natl. Acad. Sci. 21;91(13):6012-6).

Although the RNase activity of ONCONASE is relatively low, it is accepted that the enzymatic and cytotoxic activities thereof are associated to some degree. It is believed that the tertiary structure of A-RNases differentiate between cytotoxic and non-cytotoxic types. For example, differences between the tertiary structure of ONCONASE and RNase A are believed to be responsible for the increased cytotoxicity observed for ONCONASE. ONCONASE, unlike RNase A, contains a blocked N-terminal Glu1 residue (pyroglutamate) which is essential for both enzymatic and cytotoxic activities. This unique structure enables ONCONASE to permeate into target cells (Boix E., et al., 1996, J. Mol. Biol. 19:257(5):992-1007). In addition, in ONCONASE the Lys9 residue replaces the Gln11 residue of RNase A, which is believed to effect the structure of the active site. Furthermore, differences in the amino acid sequence of the primary structure between ONCONASE and RNase A cause topological changes at the periphery of the active site which effect the specificity thereof (Mosimann S.C., et al., 1992, Proteins 14(3):392-400).

The differences in toxicity between A-RNases are also attributed to their ability to bind RI. Bovine seminal ribonuclease (BS-RNase) is 80 % identical in its amino acid sequence to RNase A, but unlike other members of the RNase A superfamily, BS-RNase exists in a dimeric form. It has been shown that the quaternary structure of BS-RNase prevents binding by RI, thereby allowing the enzyme to retain its ribonucleolytic activity in the presence of RI (Kim et al., 1995, J. Biol. Chem. 270 No. 52:31097-31102). ONCONASE, which shares a high degree of homology with RNase A, is resistant to binding by RI. The RI-ONCONASE complex has a K_{d} at least one hundred million times less than that of the RI-RNase A complex. The lower binding affinity of ONCONASE for RI prevents effective inhibition of the ribonucleolytic activity and could explain why ONCONASE is cytotoxic at low concentrations while RNase A is not.

It is suggested that binding to cell surface receptor is the first step in ONCONASE cytotoxicity. Nothing is known about the nature of ONCONASE receptors on mammalian cell surfaces. ONCONASE may bind to cell surface carbohydrates as in the case of ricin, or it may bind to receptors originally developed for physiologically imported molecules like polypeptide hormones (Wu Y, et al., 1993, J. Biol. Chem. 15;268(14):10686-93). In mice, ONCONASE was eliminated from the kidneys in a rate 50-100-fold slower than did RNase A. The slower elimination rate of ONCONASE is explained as a result of its higher ability to bind to the tubular cells and/or by its resistance to proteolytic degradation. The strong retention of ONCONASE in the kidneys might have clinical implications (Vasandani V.M., et al., 1996, Cancer Res. 15;56(18):4180-6). ONCONASE may also bind to Purkinjie cells EDN receptors (Mosimann S.C., et al., 1996, J. Mol. Biol. 26; 260(4):540-52). The specificity of ONCONASE is also expressed in its tRNA preference. In rabbit reticulocyte lysate and in *Xenopus* oocytes it was discovered that ONCONASE inhibits protein synthesis via tRNA, rather than via rRNA or mRNA degradation. In contrast, RNase A degrades mostly rRNA and mRNA (Lin J.J., et al., 1994, Biochem. Biophys. Res. Commun. 14; 204(1):156-62).

Treatment of susceptible tissue cultures with ONCONASE results in the accumulation of cells arrested in G 1 phase of the cell cycle, having very low level of RNA contents (Mosimann S.C., et al., 1992, Proteins 14(3):392-400). In glioma cells ONCONASE inhibited protein synthesis without a significant reduction in cell density, showing that ONCONASE is also cytotoxic to cells in addition to being cytostatic (Wu Y., et al., 1993, J. Biol. Chem. 15;268(14):10686-93). ONCONASE, combined with chemotherapeutic agents, can overcome multidrug resistance. Treatment with vincristine and ONCONASE increased the mean survival time (MST) of mice carrying vincristine resistant tumors to 66 days, compared to 44 days in mice treated with vincristine alone (Schein, C.H., 1997, Nature Biotechnol. 15:529-536). Furthermore, some chemotherapeutic agents may act in synergy with ONCONASE. In tumor cell lines of human pancreatic adenocarcinoma and human lung carcinoma treated with a combination of ONCONASE and tamoxifen (anti-estrogen), trifluoroperazine (Stelazine, calmodulin inhibitor) or lovastatin (3-hydroxyl-3-methylglutatyl coenzyme A (HMG-CoA) reductase inhibitor) a stronger growth inhibition was observed than cells treated with ONCONASE alone (Mikulski S.M., et al., 1990, Cell Tissue Kinet. 23(3):237-46). Thus, a possibility of developing combination therapy regiments with greater efficiency and/or lower toxicity is clear.

Bovine seminal RNase is a unique member of RNase A family, since it is the only RNase containing a dimmer of RNase A-like subunits linked by two disulfide bridges. In addition, it maintains allosteric regulation by both substrate and reaction products. The regulation occurs at the cyclic nucleotide hydrolysis phase. It has the ability to cleave both single- and double-stranded RNA. BS-RNase is highly cytotoxic. It displays anti-tumor effect *in vitro* on mouse leukemic cells, HeLa and human embryo lung cells, mouse neuroblastoma cells, and human fibroblasts and mouse plasmacytoma cell lines. When administrated *in vivo* to rats bearing solid carcinomas (thyroid follicular carcinoma and its lung metastases), BS-RNase induced a drastic reduction in tumor weight, with no detectable toxic effects on the treated animals (Laccetti, P. et al., 1992, Cancer Research 52:4582-4586). Artificially monomerized BS-RNase has higher ribonuclease activity but lower cytotoxicity than native dimeric BS-RNase (D'Allessio G., et al., 1991, TIBS:104-106). This, again, indicates the importance of molecular structure for the biological activity. It seems that like ONCONASE, BS-RNase binds to recognition site(s) on the surface of the target cells, prior to penetration into target cells.

In addition to being cytotoxic, BS-RNase is also immunorepressive. BS-RNase can block the proliferation of activated T cells, and prolong the survival of skin grafts transplanted into allogenetic mice. The immunorepressive activity of SB-RNase is explained by the need to protect sperm cells from the female immune system.

### T2-RNases

In plants, self-compatibility is abundant and is effective in preventing self-fertilization. Pollen carrying a particular allele at the S locus, which controls self-incompatibility, is unable to fertilize plants carrying the same S-allele. In many self-incompatible plants, especially members of *Solanaceae* and *Rosaceae,* S-RNase, a member of the T2-RNase family is secreted by the female organs. S-RNase specifically recognize self-pollen and arrest its growth in the stigma or style before fertilization occurs (Clarke, A.E. and Newbigin, E., 1993, Ann. Rev. Genet. 27:257-279) it is believed that the arrest of pollen tube growth is a direct consequence of RNA degradation, however the mode of S-RNase entrance into the tube cell is still obscure.

Members of RNase T2 family were first identified in fungi (Egami, F. and Nakamura, K. 1969, Microbial ribonucleases. Springer-Verlag, Berlin). Since, they were found in a wide variety of organisms, ranging from viruses to mammals. In particular, T2-RNases show much broader distribution than the extensively described RNase A family. However, the *in vivo* role of T2-RNases in mammalian cells is still not known.

In microorganisms, extracellular T2-RNases are generally accepted to contribute to the digestion of polyribonucleotides present in the growth medium, thereby giving rise to diffusible nutrients. They may also serve as defense agents (Egami, F. and Nakamura, K., 1969, Microbial ribonucleases. Springer-Verlag, Berlin).

In plants, T2-RNases play a role in the pollination process, by selectively limiting the elongation of pollen tubes racing towards the ovules (Roiz, L. and Shoseyov, O., 1995, Int. J. Plant Sci. 156:37-41, Roiz L. et al., 1995, Physiol. Plant. 94:585-590). To date, the mechanism by which these RNases affect pollen tubes is unclear.

Thus, there exist few examples of cytotoxic ribonucleases which can be effectively used as cancer treatment agents. New ribonucleases with anti-proliferation, anti-colonization, anti-differentiation and/or anti-development activities toward mammalian cells are needed to enhance the spectrum of therapeutic agents available for treatment of human cancers, to thereby open new horizons in the field of cancer treatment.

There is thus a widely recognized need and it would be highly advantageous to have a novel ribonuclease that has potential usefulness in the treatment of tumor angiogenesis.

### SUMMARY OF THE INVENTION

The scope of the present invention is defined by the claims and any information that does not fall within the claims is provided for information only.

According to one aspect of the present disclosure there is provided a method of preventing, inhibiting and/or reversing proliferation, colonization, differentiation and/or development of abnormally proliferating cells in a subject, the method comprising the step of administering to the subject a therapeutically effective amount of a ribonuclease of the T2 family.

According to another aspect of the present disclosure there is provided a method of preventing, inhibiting and/or reversing proliferation, colonization, differentiation and/or development of abnormally proliferating cells in a subject, the method comprising the step of administering to the subject a therapeutically effective amount of a polynucleotide encoding and capable of expressing *in vivo* a recombinant ribonuclease of the T2 family.

According to yet another aspect of the present disclosure there is provided methods of (i) treating a tumor in a subject; (ii) preventing, inhibiting and/or reversing the development a tumor in a subject; (iii) preventing, inhibiting and/or reversing transformation of a benign tumor to a malignant tumor in a subject; (iv) preventing, inhibiting and/or reversing tumor angiogenesis in a subject; (v) reducing the number of individual tumors in a subject; (vi) reducing tumor size in a subject; (vii) reducing a number of malignant tumors in a subject; and (viii) preventing, inhibiting and/or reversing transformation of a tissue into a tumor in a subject, each of the methods is effected by administering to the subject a therapeutically effective amount of a ribonuclease of the T2 family or a therapeutically effective amount of a polynucleotide encoding and capable of expressing *in vivo* a recombinant ribonuclease of the T2 family.

According to still another aspect of the present disclosure there is provided a pharmaceutical composition comprising, as an active ingredient, a ribonuclease of the T2 family, and a pharmaceutically acceptable carrier.

According to an additional aspect of the present disclosure there is provided a pharmaceutical composition comprising, as an active ingredient, a polynucleotide encoding and capable of expressing *in vivo* a recombinant ribonuclease of the T2 family, and a pharmaceutically acceptable carrier.

According to yet an additional aspect of the present disclosure there is provided a method of preparing a medicament useful in preventing, inhibiting and/or reversing proliferation, colonization, differentiation and/or development of abnormally proliferating cells comprising the step of combining a ribonuclease of the T2 family with a pharmaceutically acceptable carrier.

According to still an additional aspect of the present disclosure there is provided a method of preparing a medicament useful in preventing, inhibiting and/or reversing proliferation, colonization, differentiation and/or development of abnormally proliferating cells comprising the step of combining a polynucleotide encoding and capable of expressing *in vivo* a recombinant ribonuclease of the T2 family with a pharmaceutically acceptable carrier.

According to further features in preferred embodiments of the invention described below, the ribonuclease of the T2 family substantially lacks ribonucleolytic activity. As used herein the phrase "substantially lacks ribonucleolytic activity" refers to (i) an inactivated ribonuclease (either natural or recombinant) of the T2 family which has 0-10 % ribonucleolytic activity as is compared to a similar, non-inactivated, ribonuclease; and/or (ii) a recombinant mutant (natural or man induced) ribonuclease of the T2 family which has 0-10 % ribonucleolytic activity as is compared to a similar, non-mutant, ribonuclease. Inactivating the ribonucleolytic activity of the ribonuclease of the T2 family may be effected by a process selected from the group consisting of boiling, autoclaving and chemically denaturing.

According to still further features in the described preferred aspects the abnormally proliferating cells are cancerous cells.

According to still further features in the described preferred embodiments the step of administering to the subject the therapeutically effective amount of the RNase of the T2 family is effected by an administration mode selected from the group consisting of oral administration, topical administration, transmucosal administration, parenteral administration, rectal administration and by inhalation.

According to still further features in the described preferred embodiments the ribonuclease of the T2 family is RNase B1.

According to still further features in the described preferred embodiments the ribonuclease of the ribonuclease T2 family is selected from the group consisting of RNase T2, RNase Rh, RNase M, RNase Trv, RNase Irp, RNase Le2, RNase Phyb, RNase LE, RNase MC, RNase CL1, RNase Bsp1, RNase RCL2, RNase Dm, RNase Oy and RNase Tp.

According to still further features in the described preferred aspects the abnormally proliferating cells are cell associated with a proliferative disorder or disease selected from the group consisting of papilloma, blastoglioma, Kaposi's sarcoma, melanoma, lung cancer, ovarian cancer, prostate cancer, squamous cell carcinoma, astrocytoma, head cancer, neck cancer, bladder cancer, breast cancer, colorectal cancer, thyroid cancer, pancreatic cancer, gastric cancer, hepatocellular carcinoma, leukemia, lymphoma, Hodgkin's disease, Burkitt's disease, arthritis, rheumatoid arthritis, diabetic retinopathy, angiogenesis, restenosis, in-stent restenosis and vascular graft restenosis.

The present invention successfully addresses the shortcomings of the presently known configurations by characterizing novel activities of ribonucleases of the T2 family useful in the inhibition of tumor angiogenesis.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

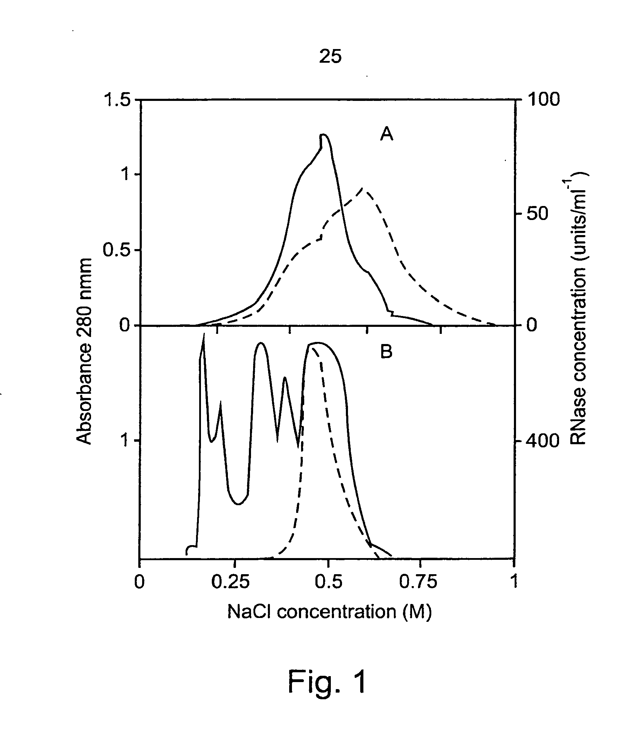

FIG. 1 is a graph representation of the absorbance and RNase activity of *Aspergillus niger* RNase B1 isolated according to the teachings of Roiz, L. and Shoseyov, O., 1995, Int. J. Plant Sci. 156:37-41. Graph A represents fractions obtained via EMD-TMAE column chromatography of a crude filtrate, while graph B represents the fraction obtained from MONO-Q column chromatography of the active fractions resulted from the EMD-TMAE chromatography of the crude filtrate. The solid line represents absorbance at 280 nm and the dashed line represents RNase activity.

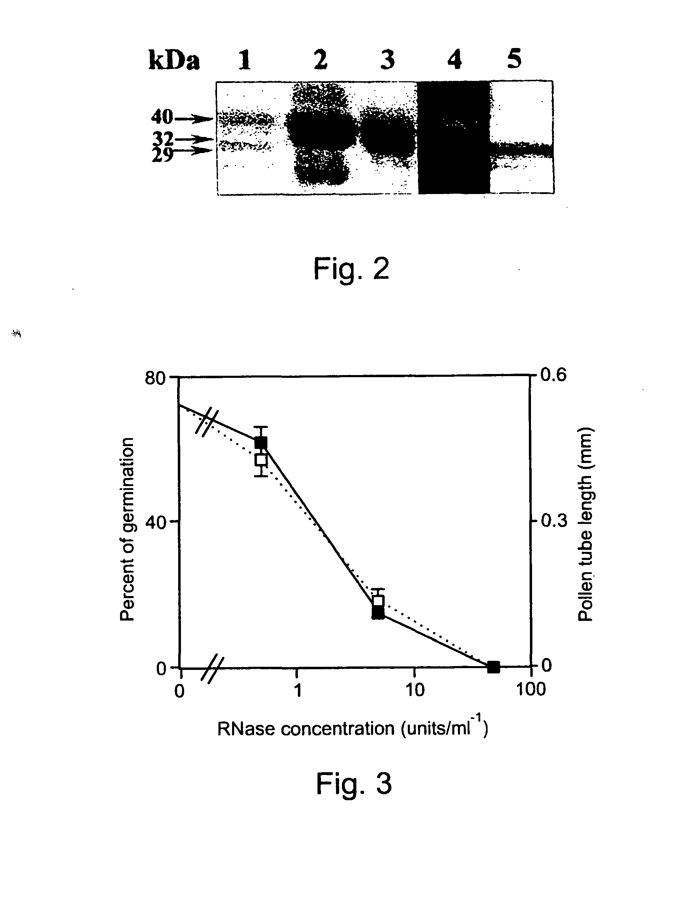

FIG. 2 is an SDS-PAGE zymogram illustrating the increase in RNase B1 protein concentration throughout the purification steps employed. Lane 1 represents the crude filtrate; lane 2 represents the eluate from the EMD-TMAE column; lane 3 represents the eluate from the MONO-Q column; lane 4 represent the eluate of lane 3 assayed *in situ* for RNase activity and stained with toluidine blue; lane 5 represents the purified RNase following deglycosilation by PNGase F. Lanes 1-3 and 5 are stained with coomassie blue.
FIG. 3 is a graph illustrating the *in vitro* effect of different concentrations of B1 RNase on peach pollen germination (solid line with black squares) and pollen tube length (dashed line with boxes).

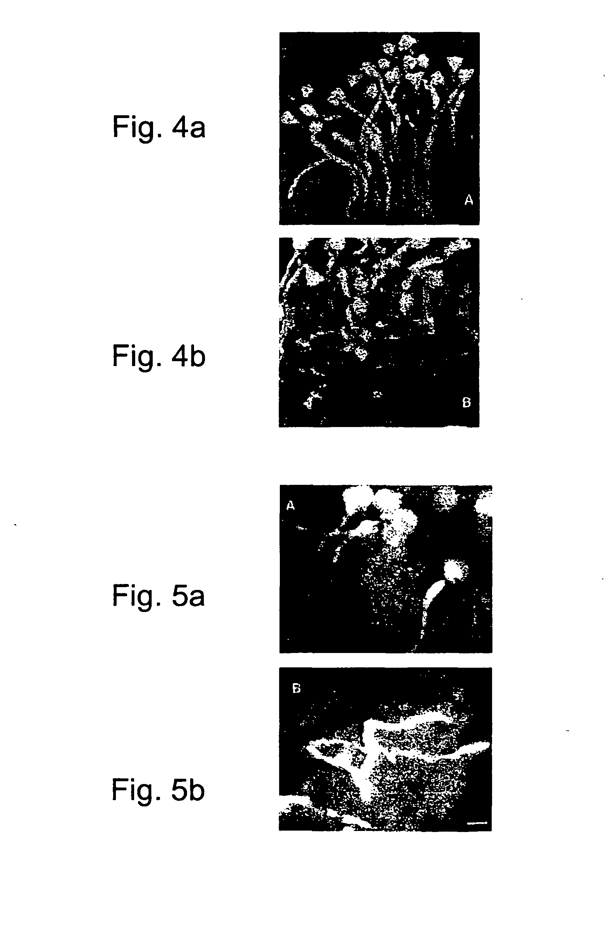

FIGs. 4a and 4b illustrate the effect of RNase B1 on peach pollen tube growth in the stigma and the upper part of the style. Figure 4a represents the control flower, while Figure 4b is a flower treated with RNase B1 before pollination. Bar = 0.2 mm
FIGs. 5a and 5b illustrate the effect of RNase B1 on pollen tube growth in the stigma of a tangerine flower. Figure 5a represents a control flower which was exposed to open pollination for 48 hours. Figure 5b represents a flower which was treated with RNase B1 prior to pollination. Bar = 0.1 mm.

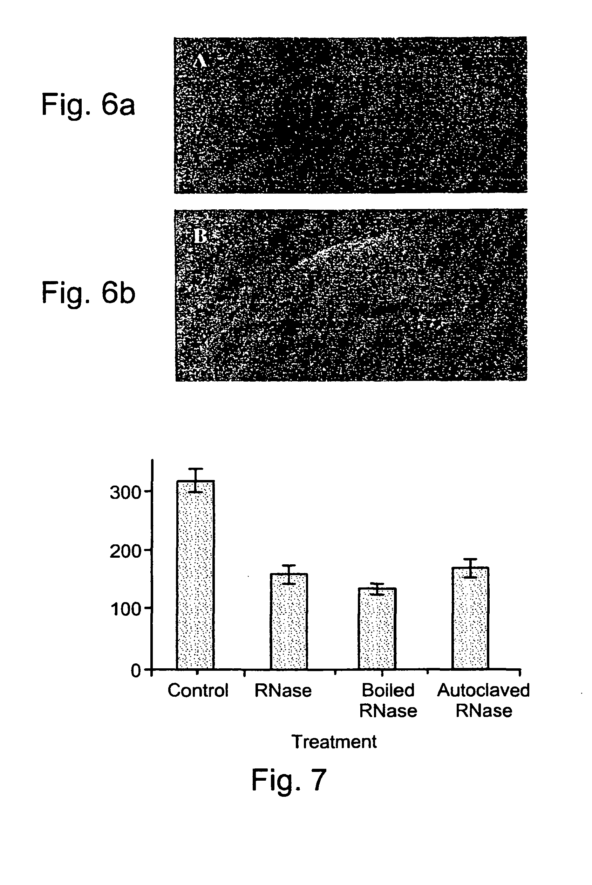

FIGs. 6a and 6b illustrate viability test conducted on nectarine seeds. Figure 6a represents a control seed produced by an untreated flower, while Figure 6b represents a seed produced by an RNase B1 treated flower. Bar = 0.3 mm.
FIG. 7 illustrates the effect of RNase B1, untreated, boiled or autoclaved, on lily cv. Osnat pollen tube length.

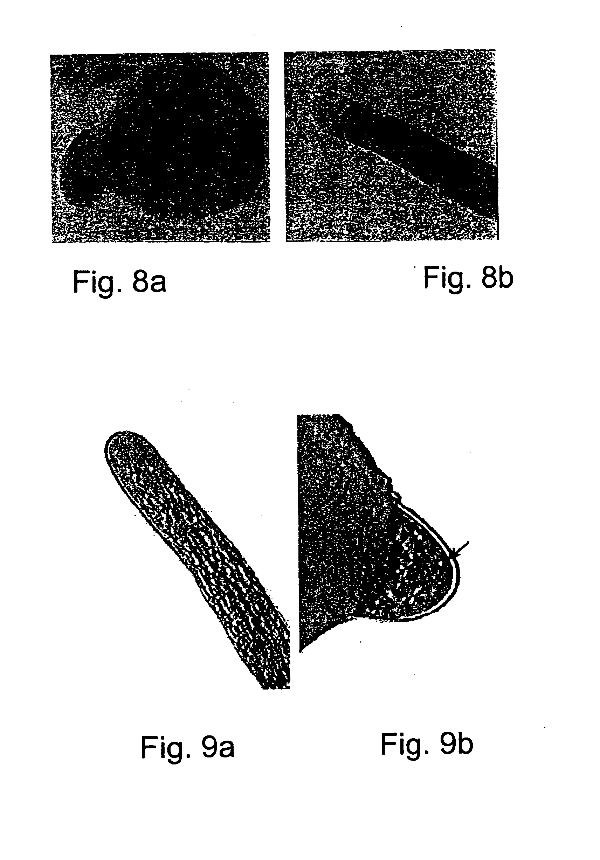

FIGs. 8a and 8b illustrate the effect of RNase B1 on lily pollen tubes growing *in vitro* and stained with IKI.
FIGs. 9a and 9b illustrate still shots captured from integrated video images showing organelle movement and localization in RNase B1 untreated (Figure 9a) and treated (Figure 9b) pollen tubes.

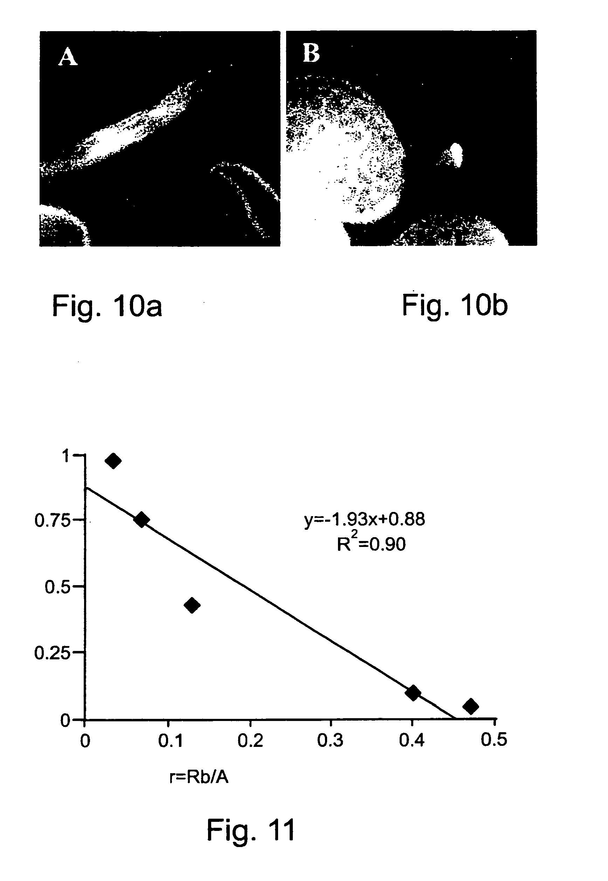

FIGs. 10a and 10b illustrate the effect of RNase B1 on actin filaments of a growing lily pollen tube. Figure 10a represents the control pollen tube whereas Figure 10b represents the RNase B1 treated pollen tube. Both pollen tubes were excised and stained with TRITC phalloidine for visualization following experimentation.
FIG. 11 is a Scatchard plot representing RNase B1 binding to actin. A - actin concentration µM), Rf - free RNase B1 concentration (µM), Rb - bound RNase B1 concentration (µM).

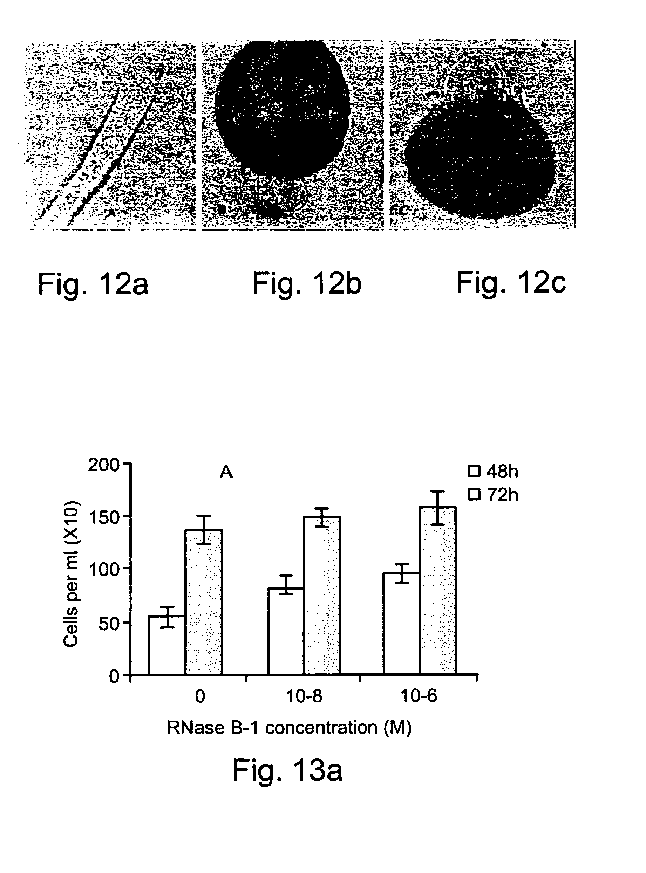

FIGs. 12a-c illustrate immunogold silver stained lily pollen tubes grown for 1 hour. Figure 12a represents a control, whereas Figures 12b and 12c are both RNase B1 treated pollen tubes. The pollen tube of Figure 12b was incubated with rabbit pre-immune serum, while the pollen tube of Figure 12c was incubated with anti-RNase B1 rabbit polyclonal antibody.

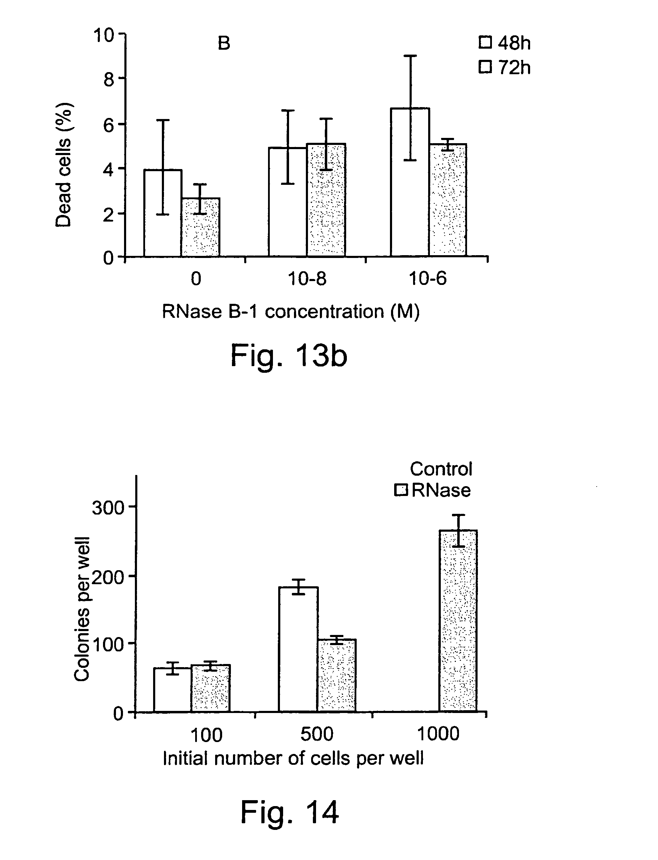

FIGs. 13a and 13b illustrate the effect of different concentrations of RNase B1 on the viability of HT29 colon cancer cells. Replicate samples of cells were grown for 48 hours or for 72 hours at 37 °C, visualized using trypan blue differential staining and counted. Figure 13a represents the total numbers of cells whereas Figure 13b represents the percent of dead cells.
FIG. 14 illustrates the effect of RNase B1 on clonogenicity of HT29 cells. Replicate samples of cells were preincubated with growth medium in the absence or presence of 10⁻⁶ M RNase B1 for 48 hours, trypsinized, washed, resuspended in RNase B1-free growth medium in serial dilutions, and plated into 96-well microtiter plates to colonize for 14 days. Colonies were counted following fixation and staining with methylene blue.

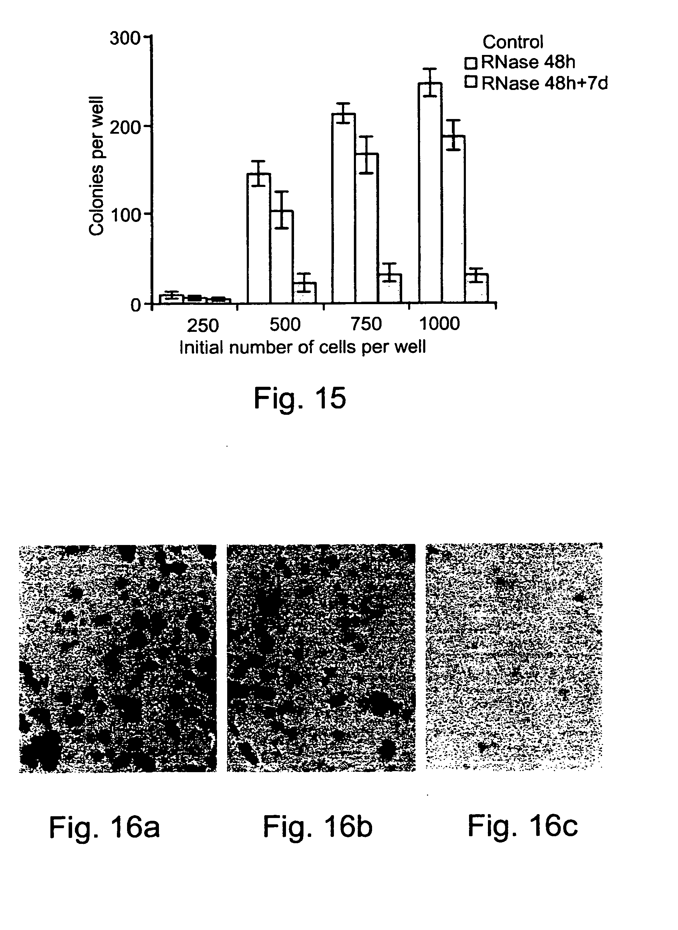

FIG. 15 illustrates the effect of exposure period to RNase B1 on the clonogenicity of HT29 cells. Replicate samples of cells were preincubated with growth medium containing 10⁻⁶ M RNase B1 for 48 hours and than let to colonize in growth medium containing the same concentration of RNase B1, or in RNase B1-free medium. Colonization was done in 96-well microtiter plates for 7 days. Each treatment contained different initial numbers of cells per well. The colonies were counted following fixation and visualization in methylene blue. Cells preincubated and colonized in RNase B1-free growth medium served as a control.
FIGs. 16a-c illustrate the effect of RNase B1 on the colonization ability of HT29 cells. Control cells (Figure 16a) were preincubated 48 hours in RNase B1-free growth medium and then trypsinized and incubated with the same growth medium in 96-microtiter plates for colonization. Figure 16b represents cells that were preincubated for 48 hours in growth medium containing 10⁻⁶ M RNase B1 and then allowed to colonize in RNase B1-free growth medium. Figure 16c represents cells that were preincubated and then colonized in growth medium containing 10⁻⁶ M RNase B1. The cell colonies were visualized using methylene blue staining.

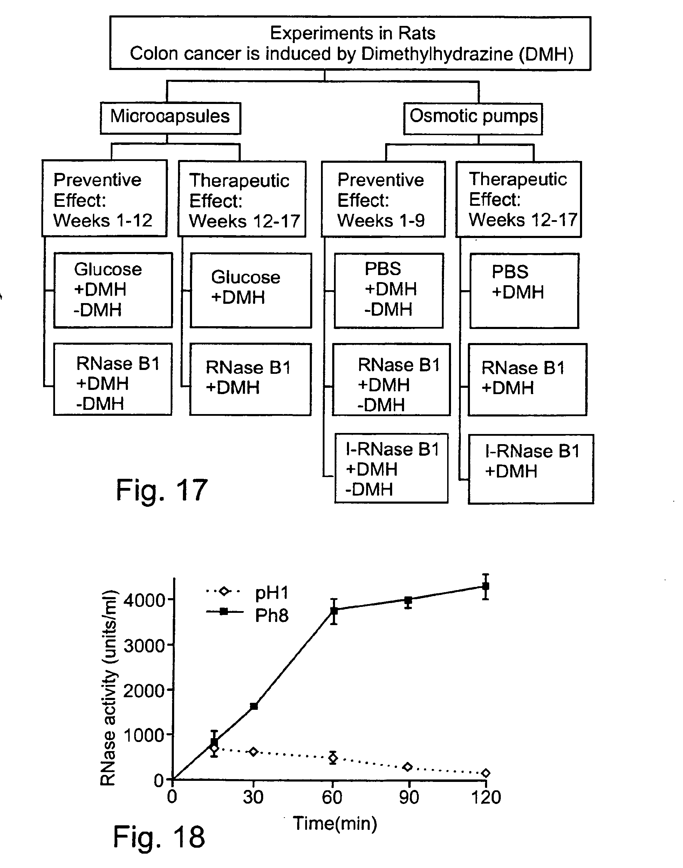

FIG. 17 is a scheme of *in vivo* experiments conducted in rats, describing the treatment for each group of 6 rats.
FIG. 18 demonstrates the effect of two different pHs on the rate of RNase B1 release from CAP microcapsules. Microcapsules containing 10 mg RNase B1 were suspended in 10 ml of 0.1 M HCl (pH 1) or 0.1 M Tris buffer (pH 8) and incubated at 37°C while stirring. Samples of upper solution were taken every 30 min for RNase activity tests.

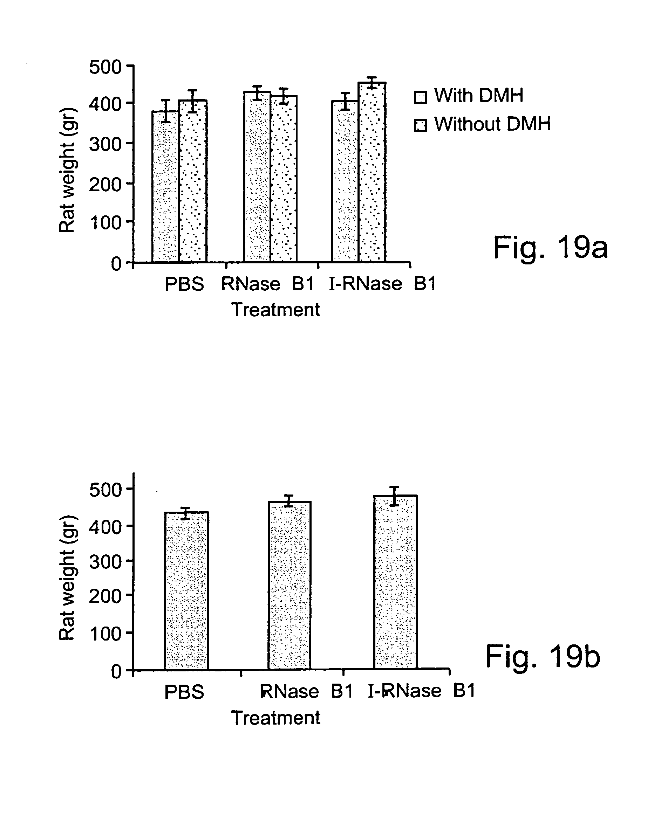

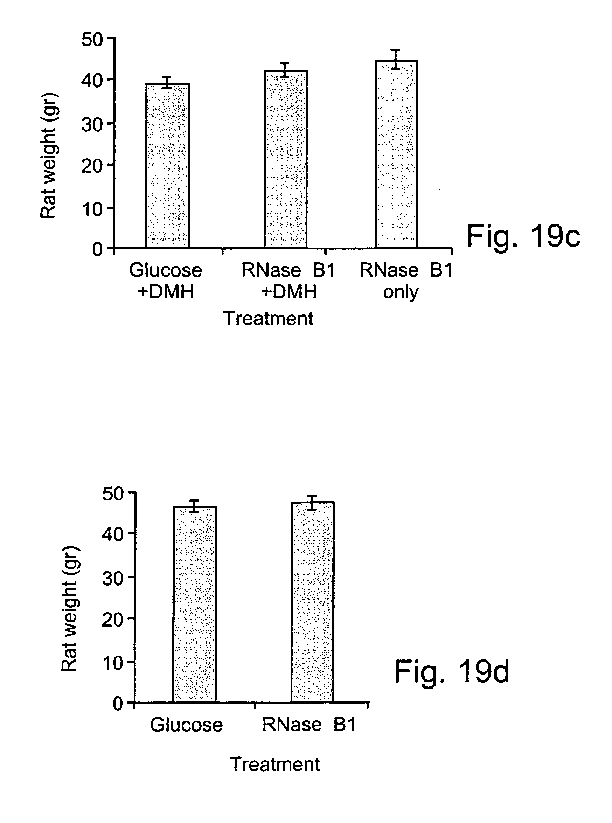

FIGs. 19a-d demonstrate the effect of RNase B1 and/or DMH on rats growth rate, as shown by body weigh at the end of each experiment. Initial rat weight was about 200 grams. n = 6. 19a - PBS, RNase B1 or I-RNase B1 was given via osmotic pumps at weeks 1-9 after first DMH injection (preventive treatment). As control rats treated as described above, but in the absence of DMH were used. 19b - PBS, RNase B1 or I-RNase B1 was given via osmotic pumps at weeks 12-17 after first DMH injection (therapeutic treatments). 19c - The rats were fed with microcapsules containing RNase B1 or glucose as a preventive treatment. As control rats that were treated with RNase B1 in the absence of DMH were used. 19d - Rats were fed with microcapsules containing RNase B1 or glucose.

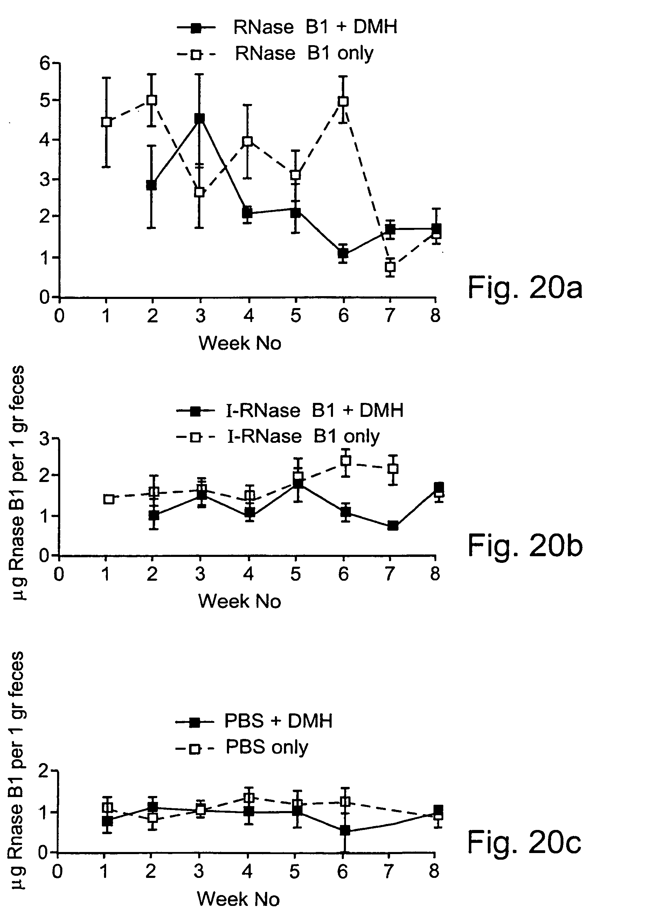

FIGs. 20a-c demonstrate RNase activity in feces of rats implanted with osmotic pumps containing RNase B1 (20a), I-RNase B1 (20b) or PBS (20c), as a preventive treatment. As control, rats were treated with RNase B1 or PBS in the absence of DMH. RNase activity was determined as described in the Examples section below.

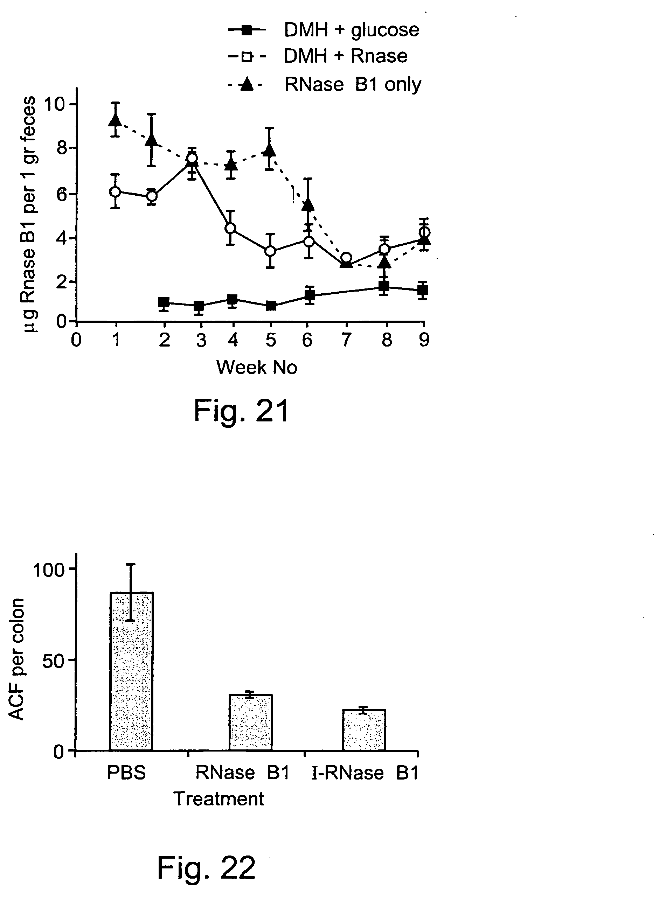

FIG. 21 demonstrates RNase activity in feces of rats fed with microcapsules containing RNase B1 or glucose as a preventive treatment. As control, rats were fed with RNase B1 or glucose in the absence of DMH. RNase activity was determined as described in the Examples section below.
FIG. 22 show the number of aberrant crypt foci (ACF) in distal colon (5 cm) of rats implanted with osmotic pumps as a preventive treatment (n = 6).

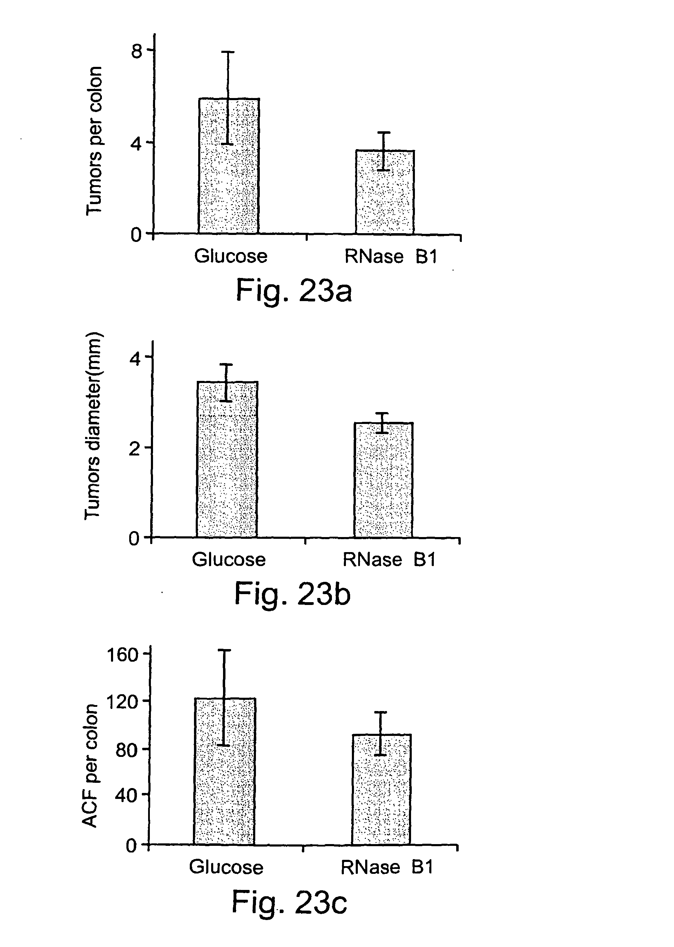

FIG. 23a-c demonstrate the effect of RNase B1 on different parameters examined in the distal (5 cm) colon of rats fed with microencapsulated RNase B1 or glucose, as a preventive treatment (n =6). 23a - number of tumors per colon; 23b - tumor size; 23c - ACF per colon.

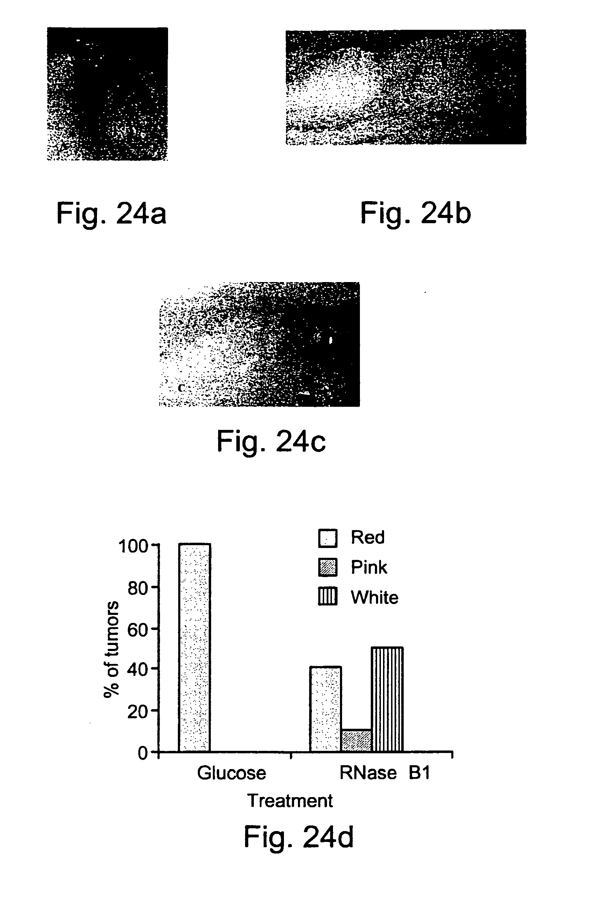

FIGs. 24a-d demonstrate different types of tumors, as photographed at the inner mucosal surface 1 hour after excision. 24a - red tumors; 24b - white tumors. 24c - a pink tumor and a red tumor; 24d - distribution of three types of tumors in rats fed with microcapsules containing glucose or RNase B1, as a preventive treatment.

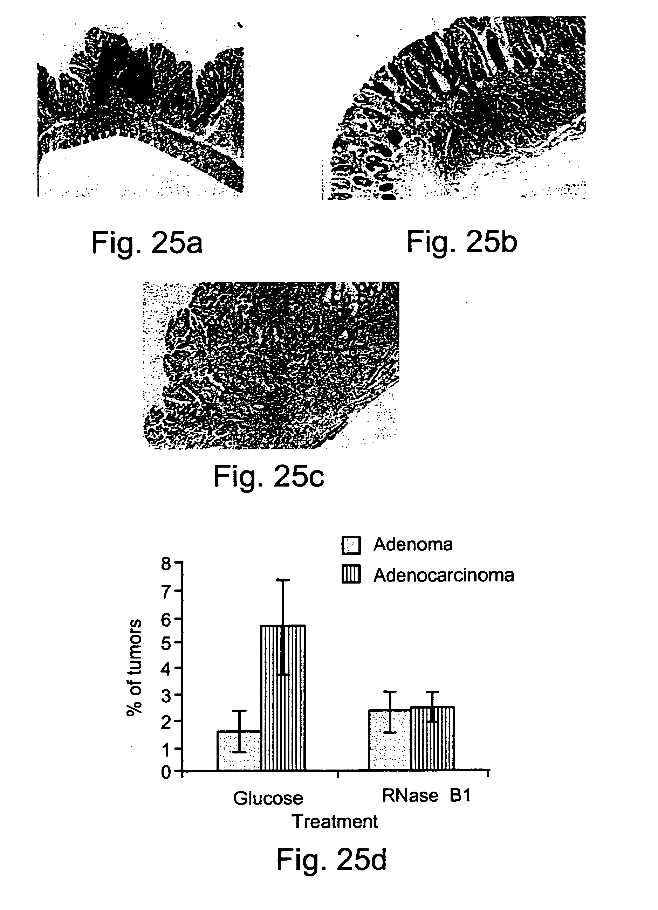

FIGs. 25a-d show histopathological examination of tumors stained with Mayer's heamatoxylin and martius-yellow. 25a - an adenoma or adenopapilloma - a benign tumor; 25b - adenocarcinoma, in which mucosal cells penetrated beneath the sub-mucosa; 25c - a well-developed adenocarcinoma, in which tissue arrangement is entirely interrupted; 25d - distribution pattern of adenoma and adenocarcinoma types of tumors in colons of rat treated with encapsulated glucose or RNase B1, as a preventive treatment.

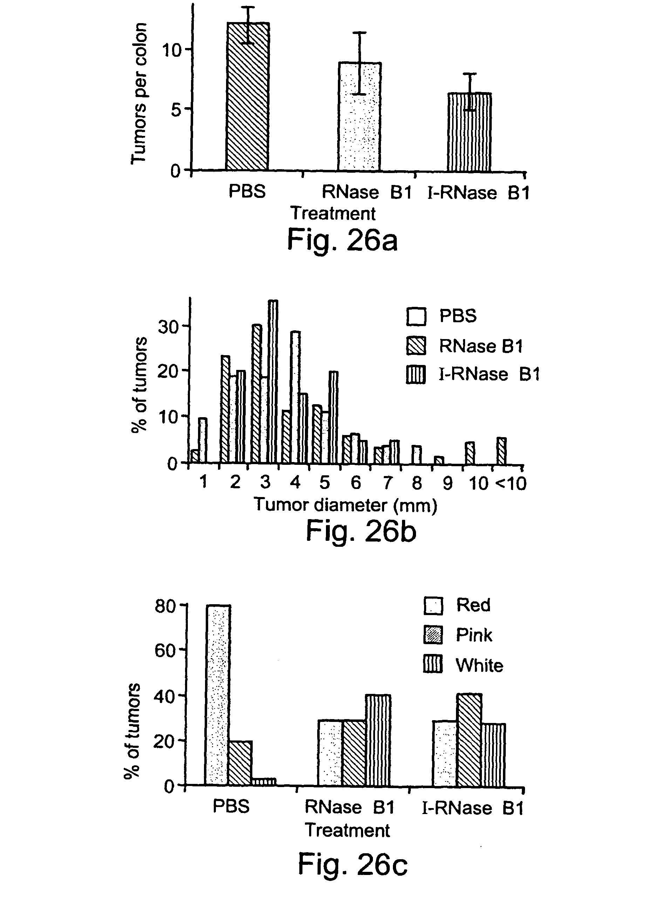

FIGs. 26a-c demonstrate the effect of RNase B1 on different parameters examined in the distal (5 cm) colon of rats treated with osmotic pumps containing PBS, RNase B1 or I-RNase B1 as a therapeutic treatment. 26a - number of tumors per colon; 26b - distribution of tumors according to size; 26c - distribution of tumors according to color, indicating angiogenesis.

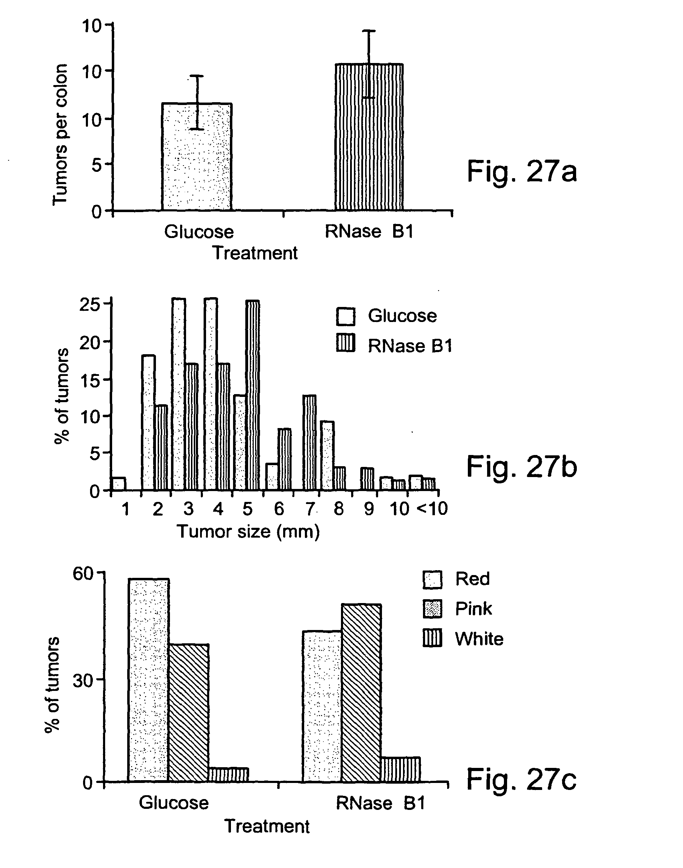

FIGs. 27a-c demonstrate the effect of RNase B1 on different parameters examined in the distal (5 cm) colon of rats fed with microencapsulated RNase B1 or glucose as therapeutic treatments. 27a - number of tumors per colon; 27b - distribution of tumors according to size; 27c - distribution of tumors according to color, indicating angiogenesis.

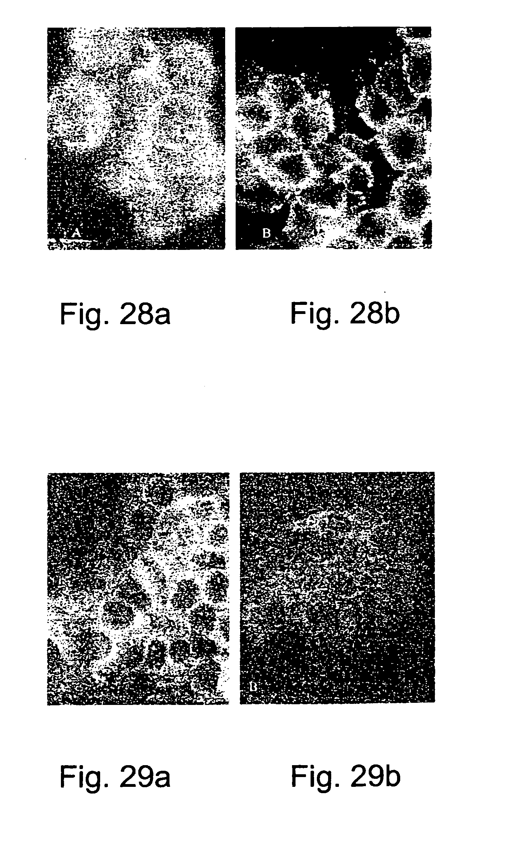

FIGs. 28a-b show human colon carcinoma HT-29 4-d cultured cells stained with TRIRC for actin. 28a - control cells; 28b - cells that were grown in the presence of 10-6 M RNase B1.
FIGs. 29a-b show human colon carcinoma HT-29 4-d cultured cells immunostained for membranal actin. 29a - control cells; 29b - cells grown in the presence of 10-6 M RNase B1.

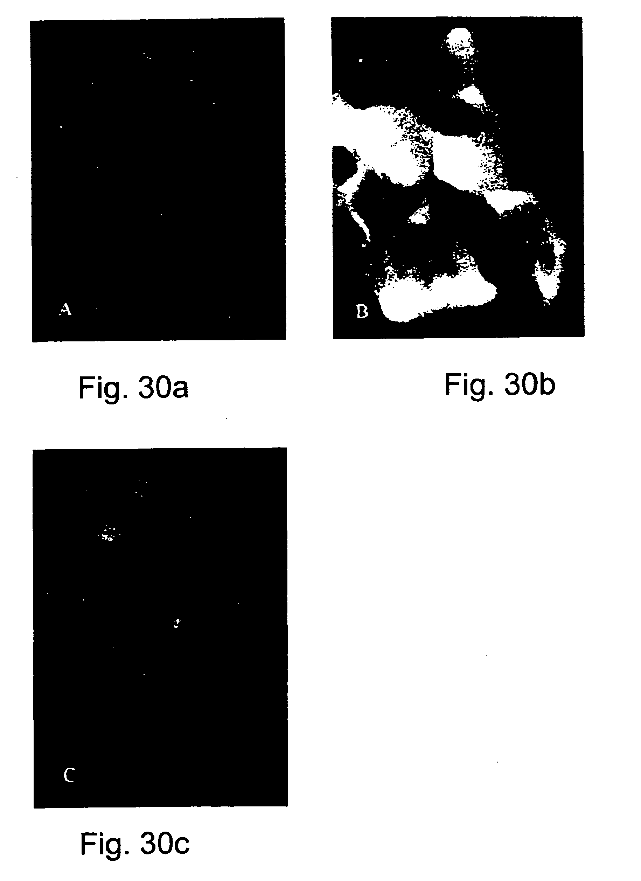

FIGs. 30a-c show human colon carcinoma HT-29 4-d cultured cells immunostained with FITC. Anti-RNase B1 was used as the primary antibody. 30a - control cells; 30b - cell that were grown in the presence of RNase B1, showing RNase B1 bound on the cell surface; 30c - pre immuned serum (PIS) was used as primary antibody.

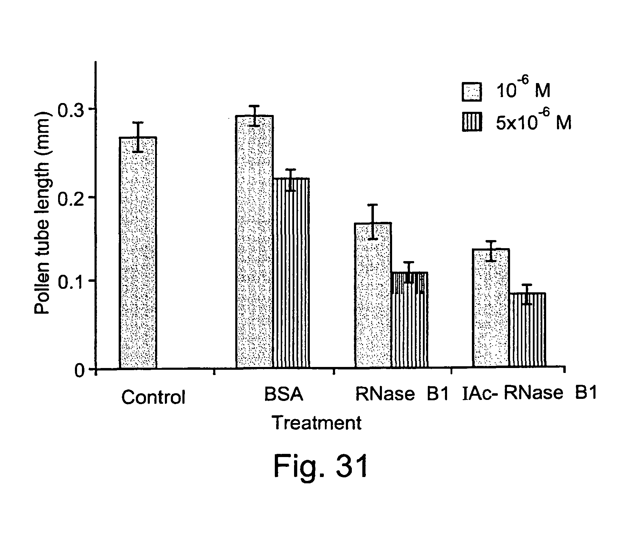

FIG. 31 demonstrates the effect of different protein treatments on lily pollen tube length. Pollen tubes were grown *in vitro* for 1 hour at 25°C as described in the Examples section that follows.

### DESCRIPTION OF THE PREFERRED EMBODIMENTS

The scope of the present invention is defined by the claims and any information that does not fall within the claims is provided for information only.

The present disclosure teaches the use of a ribonuclease of the T2 family or a polynucleotide encoding same , inhibiting tumor angiogenesis. The present disclosure further teaches pharmaceutical compositions containing, as an active ingredient, a ribonuclease of the T2 family or a polynucleotide encoding same for treating proliferative diseases or disorders in general and cancer in particular.

The use of ribonucleases with cytotoxic activity for inhibiting the proliferation of tumor cells is not new and has been demonstrated previously in the art. A ribonuclease of the A family which is commercially known as ONCONASE has been shown to inhibit cell proliferation in tumorous tissue in clinical trials. Several other RNases of the RNase A superfamily have also been demonstrated to have cytotoxic activity in addition to their ribonucleolytic activity.

Although the cytotoxicity of some ribonucleases is dependent to some extent on their ribonucleolytic activity, the level of ribonucleolytic activity does not always correlate to the level of cytotoxicity observed for ribonucleases. Furthermore, there exist several examples of ribonucleases which do not display cytotoxic activity altogether, yet function well as ribonucleases. The most known example is RNase A. In other cases, the reaction rate is scarified for more specific binding or improved function in some other capacity. For example, angigenin's active site is blocked by side chains that are not present in RNase A, rendering it 10,000-fold less active on general substrates, but more specific in cleaving ribosomal RNA. BS-RNase is a faster nuclease when it is monomeric. However, its cytotoxicity is higher, and inhibition by ribonuclease inhibitor is considerably reduced in the dimer form. Glycosilated RNase B is less active than RNase A on most substrates, while a frequently observed deamidation in BS-RNase, (asparagine 67 to isoaspartate) reduces the activity of RNase A mutants by cleaving a whole chain of H-bond structures in the protein. (reviewed in Shein, C.H. 1997. Nature Biotechnol 15: 529-536).

Ribonucleases of the T2 family are characterized by their unique molecular features. A comparison between RNase members of the A and of the T2 families is summarized below in Table 1 (Location of amino acids are after RNase A and RNase T2 in families A and T2, respectively).

**TABLE 1**

| **Feature** | **RNase A** | **RNase T2** |
|---|---|---|
| **Molecular mass** | 11-14 kDa (with the exception of BS-RNase) | 24-36 kDa. |
| **Optimal temperature for RNase activity:** | 37°C | 50-60°C |
| **Optimal pH for RNase activity:** | 6.5-8 | 3.5-5 |
| | Not glycosilated | 12-25% of the total molecular mass |
| **Base specificity:** | Pyrimidine base-specific. | Non specific with adenylic acid preferential. |
| **Disulfide bonds:** | Four: Common: Cys28-84, Cys40-96, Cys58-110. In pancreatic RNases the fourth S-S bond is located between Cys65-72, forming a loop containing Glu69 and Asn71, which are part the nucleotide-binding site. In ONCONASE and bullfrog lectin Cys87-Cys104 form a COOH-terminal loop, which is located near the active site. Angiogenins have only 3 disulfide bonds. | Five: Cys3-20, Cys10-53, Cys19-120, Cys63-112 and Cys182-213. |
| **Mechanis of RNase activity:** | Active site Two steps in RNA cleavage (i) His12 acts as a general base and removes a proton from the 2'-hydroxyl group of the RNA. His 119 acts as a general acid, donating a proton from the 5' O of the leaving nucleotide. (ii) The resultant 2'3'-cyclic nucleotides are hydrolyzed, with the roles of His12 and His119 reversed. Lys41 stabilizes the pentavalent transition state. Substrate binding sites: GLn11 and Phe120 form hydrogen bonds with the substrate. In ONCONASE and bullfrog lectine Glu11 forms H-bond with the phosphate of the substrate. Gln96, Asn71, Glu111, of which Asn71 is the most conserved, might catalyze RNA cleavage. | Active site RNA catalysis is similar to RNase A. His46 and His109 function as general acid and base catalysts. Glu 105 and Lys108 might plays a role in polarizing the P=O bond of the substrate or in stabilizing the pentacovalent transition state. Substrate binding sites: His104 (In plants it is Tyr or Asp) might act as the phosphate receptor of the substrate. There are two recognition sites: The major (B1) site contains Tyr57, Trp49 and Asp51. Asp51 is responsible for the adenine base recognition. A minor (B2) site contains Phe101, Gln95, Asn94, Ser93, Pro92 and Gln32. |

The ribonucleases of the T2 family have been identified in numerous microorganisms, as well as in plants, in which they play an active role in the pollination process, by selectively limiting the elongation of pollen tubes racing towards the ovules.

As uncovered by the inventors of the present invention and as is further detailed hereinbelow in Examples 1, 2 and 6, RNase B1, a T2 ribonuclease, either ribonucleolytically active or ribonucleolytically non-active, specifically binds to actin in elongating pollen tubes to thereby inhibit the elongation of pollen tubes and also to actin of mammalian cells.

Actin is known to form filaments which are essential cytoskeletal components of cells, active in both maintaining cellular structure and in supporting intracellular transport of organelles. As a result, actin filaments participate in many cellular processes throughout the life cycle of normal and abnormal cells, including proliferation, colonization, differentiation, transformation and other developmental aspects including tissue formation. Numerous studies have shown that actin also participates in various cellular processes controlling generation of cancer cells (Jordan, M.A. & Wilson, L. 1998. Curr. Opin. Cell Biol. 10:123-130; Jammy, P.A. & Chaponnier, C. 1995. Curr. Opin. Cell Biol. 7:111-117: Sigmond, S.H. 1996. Curr. Opin. Cell Biol. 8:66-73; Tapon, N. et al. 1997. Curr. Opin. Cell Biol. 9:86-92). Thus, for example, actin filaments participates in abnormal cell proliferation (Assoian, R.K. & Zhu, X. 1997. Curr. Opin. Cell Biol. 9:93-98). Malignant cells were found more sensitive to cytochalasin B than normal cells (Hemstreet G.P. et al. 1996. J. Cell Biochem. 25S:197-204).

Since actin is a highly conserved protein, maintaining a high level of homology between evolutionary distant organisms it was hypothesized that the actin binding activity of RNase B1, which inhibits pollen tube elongation can be utilized, without being limited by this theory, to specifically bind actin of mammalian cells, to thereby inhibit the proliferation, colonization, differentiation and/or development thereof.

While reducing the present disclosure to practice and as is further described in Example 2 and 5 of the Examples section, exogenous RNase B1 specifically binds to membranal actin and causes a cellular actin network disorder. As is shown in Examples 3-5, the effect of RNase B1 on mammalian cancer cells was further investigated *in vitro* and *in vivo.* As clearly demonstrated therein, RNase B1 (i) substantially decreases proliferation and/or colonization of adenocarcinoma cells grown in culture; and (ii) reduces the number of aberrant crypt foci (ACF), reduces the number and size of tumors, interferes with tumor angiogenesis, reduces the malignancy of tumors and the transition from adenoma to adenocarcinoma in a colon carcinoma rat model, in a preventive and/or therapeutic manner, while having no apparent side effects on healthy tissue in the colon or elsewhere.

One or more ribonucleases of the T2 family are collectively referred to herein as T2-RNase. Similarly, one or more polynucleotides encoding one or more ribonucleases of the T2 family are collectively referred to herein as a polynucleotide encoding a T2-RNase (or same).

Thus, according to one aspect of the present invention there is provided a method of inhibiting tumor angiogenesis. The method according to this aspect of the present invention is effected by administering to the subject a therapeutically effective amount of a ribonuclease of the T2 family or of a polynucleotide encoding and capable of expressing *in vivo* a recombinant ribonuclease of the T2 family, either *per se* or as an active ingredient of a pharmaceutical composition.

Thus, according to another aspect of the present invention there is provided a pharmaceutical composition comprising, as an active ingredient, a ribonuclease of the T2 family a polynucleotide encoding and capable of expressing *in vivo* a recombinant ribonuclease of the T2 family, and a pharmaceutically acceptable carrier.

According to yet another aspect of the present invention there is provided a method of preparing a medicament useful in preventing, inhibiting tumor angiogenesis comprising the step of combining a ribonuclease of the T2 family or a polynucleotide encoding and capable of expressing *in vivo* a recombinant ribonuclease of the T2 family, with a pharmaceutically acceptable carrier.

The medicament is preferably identified as providing a treatment for a specified proliferative disorder or disease, such as a specified cancer. Such an identification can be made in print on, for example, a container containing the medicament or on a leaflet, as is well known in the art.

The method and pharmaceutical composition of the present disclosure can be used for, for example, (i) treating a tumor in a subject; (ii) preventing, inhibiting and/or reversing the development a tumor in a subject; (iii) preventing, inhibiting and/or reversing transformation of a benign tumor to a malignant tumor in a subject; (iv) preventing, inhibiting and/or reversing tumor angiogenesis in a subject; (v) reducing the number of individual tumors in a subject; (vi) reducing tumor size in a subject; (vii) reducing a number of malignant tumors in a subject; and/or (viii) preventing, inhibiting and/or reversing transformation of a tissue into a tumor in a subject.

The T2-RNase can be derived from a native source, as is further exemplified in Example 1 that follows, or alternatively, it can be produced as a recombinant protein using an appropriate polynucleotide (see Table 2 below and the following descriptions) and expression system. Expressing and purifying recombinant proteins is well known in the art and can be effected by any one of a plurality of alternative techniques described in detail in any one of a number of text books and laboratory protocol books, including, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998).

**TABLE 2**

| **Source** | | **Name (Prot)** | **Name (Gene)** | **Reference(s)** | **GeneBank Accession No.** |
|---|---|---|---|---|---|
| | | | | | |
| ***Bacteria*** | *Aeromonas hydrophila* | | Locus RNI | Favre,D. et al. 1993. J.Bacteriol. 175:3710-3722. | Q07465 |
| | *Haemophilus influenzae* | Rnase HI0526 | Locus RN26 | Fleischmann, R.D., et al. 1995. Science 269:496-512. | P44012 |
| | *Escherichia coli* | Rnase I | Locus RNI | Meador,J. III. & Kennell,D. 1990. Gene 95:1-7. Oshima, T., et al. 1996. DNA Res. 3:137-155. Henikoff, S. & Henikoff, J.G. 1994. Genomics 19:97-107. | P21338 |
| | *Aspergillus oryzae* | Rnase T2 | rnt B | Kawata Y. et al. 1988. Eur J. Biochem 176(3):683-97. Kawata Y. et al. 1990. Eur J. Biochem 187:255-62. Ozeki K, et al. 1991. Curr Genet. 19:367-73. | P10281 |
| ***Fungi*** | *Rhisopus niveus* | Rnase Rh | | Horiuchi,H. et al. 1988. J. Biochem. 103:408-418. Kurihara,H. et al. 1992. FEBS Lett. 306:189-192. Kurihara,H. et al. 1996. J. Mol. Biol. 255:310-320. Ohgi, K. et al. 1991. J. Biochem. 109:776-785. | P08056 |
| | *Trichoderma viride* | Rnase Trv | | Inada,Y. et al. 1991. J. Biochem. 110 (6), 896-904. | P24657 |
| | *Lentinula edodes* (shiitake mushroom) | Rnase Irp | | Kobayashi, H. et al. 1992. Biosci. Biotechnol. Biochem. 56:2003-2010. | AAB24971 |
| | *L. edodes* | Rnase Le2 | | Kobayashi,H. et al. 1992. Biosci. Biotechnol. Biochem. 56:2003-2010. Shimada,H. et al. 1991. Agric. Biol. Chem. 55:1167-1169. | P81296 |
| | *Irpex lacteus* | Rnase Irp1 | | Watanabe,H., et al. 1995. Biosci. Biotechnol. Biochem. 59:2097-2103. | AAB35880 |
| | *Physarum polycephlum* | Rnase Phyb | | Inokuchi,N. et al. 1993. J. Biochem. 113:425-432. | P81477 |
| ***Plants*** | *Arabidopsis thaliana* | RNS2 | Locus RNS2 | Green,P.J. 1993. Proc. Natl. Acad. Sci. U.S.A. 90:5118-5122. | P42814 |
| | *A. thaliana* | Rnase 3 | Locus RNS3 | Bariola,P.A., et al. 1994. Plant J. 6:673-685. | P42815 |
| | *A. thaliana* | Rnase 1 | Locus RNS1 | Bariola,P.A., et al. 1994. Plant J. 6:673-685. | P42813 |
| | *Lycopersicon esculentum* (cultured tomato) | Rnase LE | RNALE | Kock,M. et al. 1995. Plant Mol. Biol. 27:477-485. Jost,W. et al. 1991. Eur. J. Biochem. 198:1-6. | P80022 |
| | *L. esculentum* | Rnase LX | RNLX | Kock,M., et al. 1995. Plant Mol. Biol. 27:477-485. Loffler,A., et al. 1993. Eur. J. Biochem. 214:627-633. | P80196 |
| | *Nicotiana alata (*tobacco*)* | S-RNase | S | Anderson ,M.A., et al. 1986. Nature 321:38-44. Matton,D.P. et al. 1995. Plant Mol. Biol. 28:847-858. McClure ,B.A. et al. 1989. Nature 342:95-97. | P04002 |
| | *Malus domestica* (apple tree) | S-RNases | S | Sassa,H., et al. 1996. Mol. Gen. Genet. 250:547-557. | |
| | *Pyrus pyrifolia* (Japanese pear) | S-RNases | S | Norioka,N., et al. 1996. J. Biochem. 120 ;335-345. | |
| | *Momordica charantia* (bitter gourd) | RNase MC | Locus RNMC | Blaxter,M.L., et al. 1996. Mol. Biochem. Parasitol. 77:77-93. Ide,H. et al. 1991. FEBS Lett. 284:161-164. Ide,H. et al. 1991. FEBS Lett. 289:126. | P23540 |
| *Animals* | *Gallus gallus* (chicken) | RNase CL1 | | Uchida,T. et al. 1996. Biosci. Biotechnol. Biochem. 60:1982-1988. | JC5126 |
| | *Rana catesbeiana* (bull frog) | RNase RCL2 | | Yagi,H. et al. 1995. Biol. Pharm. Bull. 18:219-222. Liao,Y.D. et al. 1996. Protein Expr Purif. 7:194-202. Liao YD, et al. 1994. Eur J Biochem. 222:215-20. Liao,Y.D. et al. 1998. J. Biol. Chem. 273: 6395-401 | PC2347 |
| | *Drosophyla melanogaster* | RNase DM | DmRNase | Lankenau,D.H. et al. 1990. Chromosoma 99:111-117. Hime,G., et al. 1995. Gene 158:203-207. | X15066 |
| | *Crassostera gigus* (pacific oyster.) | RNase Oy | Locus JX0295 | Watanabe,H. et al. 1993. J. Biochem. 114:800-807. | JX029 |
| | *Todarodes pasificus* (Japanese flying squid) | RNase Tp | | Kusano, A. et al. 1998. Biosci. Biotechnol. Biochem. 62:87-94. | PMID 9501521 |
| | *Homo sapiens* | RNase 6 precurs. | RNase6PL | Trubia, M. et al. 1997. Genomics 42:342-344. | NP003721 |

For some applications it may be beneficial to use a ribonuclease which substantially lacks ribonucleolytic activity, which may have or cause undesired side effects. As used herein the phrase "substantially lacks ribonucleolytic activity" refers to (i) an inactivated ribonuclease (either natural or recombinant) of the T2 family which has 0-10 % ribonucleolytic activity as is compared to a similar, non-inactivated, ribonuclease; and/or (ii) a recombinant mutant (natural isolate or man induced) ribonuclease of the T2 family which has 0-10 % ribonucleolytic activity as is compared to a similar, non-mutant, ribonuclease. Inactivating the ribonucleolytic activity of the ribonuclease of the T2 family may be effected by a process selected from the group consisting of boiling, autoclaving and chemically denaturing or inactivating.

As is further detailed in Examples 2 and 6 below, it has been shown by the inventors that the anti-proliferation, anti-colonization, anti-differentiation and/or anti-development activities of RNase B1 are not dependent on its ribonucleolytic activity, as boiled, autoclaved and chemically inactivated (acetylated) RNase B1, which has little (10 %) or substantially no (0-10 %) ribonucleolytic activity retained substantially all of its anti-proliferation, anti-colonization, anti-differentiation and/or anti-development activities.

Thus, a T2-RNase protein according to the present invention can be utilized both in a native, ribonucleolytic active, form, or, alternatively, in a silent, or repressed ribonucleolytic form, having no (0 %) or little (up to 10 %) ribonucleolytic activity, yet which retains its other activities. As such, the term "T2-RNases" is meant to encompass all the anti-proliferation, anti-colonization, anti-differentiation and/or anti-development forms of the protein, regardless of other activities thereof.

It will be appreciated that utilizing a T2-RNase, either directly or expressed from a polynucleotide, which displays a desired activity and yet is devoid of, or repressed in, ribonucleolytic activity is particularly advantageous since ribonucleolytic activity can produce undesired side effects in a subject.

A polypeptide representing the amino acid sequence of a T2-RNase as defined herein can be produced by any one of several methods well known in the art. For example the polypeptide can be produced synthetically by standard peptide synthesis techniques, for example using either standard 9-fluorenylmethoxycarbonyl (F-Moc) chemistry (see, for example, Atherton, E. and Sheppard, R. C. 1985, J. Chem. Soc. Chem. Comm. 165) or standard butyloxycarbonate (T-Boc) chemistry, although it is noted that, more recently, the fluorenylmethoxycarbonyl (Fmoc)/tert-butyl system, developed by Sheppard has found increasingly wide application (Sheppard, R. C.1986 Science Tools, The LKB Journal 33, 9).

Alternatively, a T2-RNase protein can also be isolated and purified by methods well known in the art from organisms known to express this protein. Such organisms include, for example, *Aeromonas hydrophila, Haemophilus influenzae, Escherichia coli, Aspergillus oryzae, Aspergillus phoenicis, Rhisopus niveus, Trichoderma viride, Lentinula edodes, Irpex lacteus; Physarum polycephlum, Arabidopsis thaliana, Lycopersicon esculentum, Nicotiana alata, Malus domestica, Pyrus pyrifolia, Momordica charantia, Gallus gallus, Rana catesbeiana, Drosophyla melanogaster, Crassostera gigus, Todarodes pasificus and Homo sapiens.* It is, however, anticipated that other organisms yet not known to produce T2-RNase, once uncovered as such, could also be used as a source for T2-RNase according to the present invention.

Alternatively and preferably a T2-RNase protein can be recombinantly produced by expressing a polynucleotide encoding same, using an appropriate expression vector system. Preferably, an expression system is selected which provides suitable post translational modifications. Suitable expression vector systems include, but are not limited to, mammalian cells infected with a virus (e.g., adenovirus, retrovirus, herpes simplex virus, avipox virus); insect cells infected with a virus (e.g., baculovirus); genetically modified plants or plant cells transformed with a plasmid, a plant virus or an *Agrobacterium;* transformed microorganisms such as yeasts containing yeast vectors, or bacteria transformed with bacteriophage DNA, plasmid DNA, or cosmid DNA. The expression controlling elements of vectors vary in their strengths and specifications depending on the host-vector system utilized, any one of a number of suitable transcription and translation elements may be used. A recombinantlly produced T2-RNase may be purified from host cells by affinity chromatography, electrophoresis, high-performance liquid chromatography (HPLC), immunopercipitation, sedimentation or any other method known to the art.

A purified T2-RNase can be used to prepare a medicament according to the present invention by means of conventional mixing, dissolving, granulating, dragee-making, levigating, emulsifying, encapsulating, entrapping or lyophilizing processes with the addition of the appropriate pharmaceutically acceptable carriers and/or excipients or alternatively it can be linked to appropriate delivery vehicles as described hereinabove.

A polynucleotide according to the present invention can encode a native T2-RNase protein, which term in this context to describe a T2-RNase having both anti-proliferative and ribonucleolytic activities, or alternatively, a polynucleotide according to the present invention can encode a silent or repressed T2-RNase mutant, having no or little ribonucleolytic activity, to be expressed (e.g., transcribed and translated) *in vivo* into a protein which is substantially free of ribonucleolytic activity.

As such, the term "polynucleotide" when used herein in context of T2-RNases in general, or in context of any specific T2-RNase, refers to any polynucleotide sequence which encodes a T2-RNase active in preventing, inhibiting and/or reversing proliferation, colonization, differentiation and/or development of abnormally proliferating cells, either having or substantially devoid of ribonucleolytic activity. Polynucleotides encoding a T2-RNase devoid of ribonucleolytic activity can be obtained using known molecular biology techniques, such as random mutagenesis, site-directed mutagenesis and enhanced evolution techniques. Site directed mutagenesis can be readily employed because the amino acid residues essential for the ribonucleolytic activity of T2-RNases have been recognized (see Kusano et al., 1998. Biosci. Biothechnol. Biochem. 62:87-94, and Table 2 above).

Thus, the present invention can be used for treating conditions, syndromes or diseases characterized by abnormally proliferating cells, such as cancerous or other cells, such as, but not limited to, papilloma, blastoglioma, Kaposi's sarcoma, melanoma, lung cancer, ovarian cancer, prostate cancer, squamous cell carcinoma, astrocytoma, head cancer, neck cancer, bladder cancer, breast cancer, colorectal cancer, thyroid cancer, pancreatic cancer, gastric cancer, hepatocellular carcinoma, leukemia, lymphoma, Hodgkin's disease, Burkitt's disease, arthritis, rheumatoid arthritis, diabetic retinopathy, angiogenesis, restenosis, in-stent restenosis and vascular graft restenosis.

As used herein the terms "cancer" or "tumor" are clinically descriptive terms which encompass a myriad of diseases characterized by cells that exhibit abnormal cellular proliferation.The term "tumor", when applied to tissue, generally refers to any abnormal tissue growth, characterized in excessive and abnormal cellular proliferation. A tumor may be "benign" and unable to spread from its original focus, or "malignant" or "metastatic" and capable of spreading beyond its anatomical site to other areas throughout the host body. The term "cancer" is an older term which is generally used to describe a malignant tumor or the disease state arising therefrom. Alternatively, the art refers to an abnormal growth as a neoplasm, and to a malignant abnormal growth as a malignant neoplasm.

Any ribonuclease of the T2 family which has the anti-angiogenesis, activities described herein and exemplified in the Examples section that follows can be used as a therapeutic agent in accordance with the teachings of the present invention. Similarly, any polynucleotide encoding a ribonuclease of the T2 family which has the anti-angiogenesis activities described herein can be used as a therapeutic agent in accordance with the teachings of the present invention. A non exhausting list of ribonucleases of the T2 family is provided in Table 2, above. As is further exemplified by the Examples that follow, RNase B1, which is a member of the T2 family, has anti-angiogenesis as determined by *in vivo* and *in vitro* assays. In addition, RNase B1 is shown to bind to actin even when treated so as to render it free of ribonuclease activity. Thus, the present invention provides three different assays with which one of ordinary skills in the art could test a given ribonuclease for its anti-angiogenesis activities, these are an in vitro assay for determining the effect of the tested ribonuclease on cancerous cells, in vivo assay for determining the effect of the tested ribonuclease on tumor development, and another in vitro assay for determining the ability of the tested ribonuclease to bind to cellular and/or free actin. Without limiting the present invention by any theory, it is believed that an ability of a ribonuclease to bind to actin is indicative that such a ribonuclease has anti-proliferation, anti-colonization, anti-differentiation and/or anti-development activities.

A ribonuclease according to the present invention can be administered to an organism, such as a human being or any other mammal, *per se,* or in a pharmaceutical composition where it is mixed with suitable carriers or excipients.

As used herein a "pharmaceutical composition" or "medicament" refers to a preparation of one or more of the ribonucleases or polynucleotides encoding same as described herein" with other chemical components such as physiologically suitable carriers and excipients. The purpose of a pharmaceutical composition is to facilitate administration of a compound to an organism.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

Pharmaceutical compositions may also include one or more additional active ingredients, such as, but not limited to, anti inflammatory agents, antimicrobial agents, anesthetics and the like in addition to the main active ingredient.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active compounds into preparations which can be used pharmaceutically. Proper formulation is dependent upon the route of administration chosen.

Thus, to effect administration the pharmaceutical composition of the present invention includes a suitable pharmaceutical carrier and an effective amount of a T2-RNase or a polynucleotide encoding same, and is administered, for example, topically, intraocularly, parenterally, orally, intranasally, intravenously, intramuscularly, subcutaneously or by any other effective means via methods well known in the art.

For intravenously, intramuscularly or subcutaneously injection, a T2-RNase or a polynucleotide encoding same may be formulated in aqueous solutions, preferably in physiologically compatible buffers such as Hank's solution, Ringer's solution, or physiological saline buffer. For example, a physiologically appropriate solution containing an effective amount of a T2-RNase or a polynucleotide encoding same can be administered systemically into the blood circulation to treat a cancer or tumor which cannot be directly reached or anatomically isolated. A physiologically appropriate solution containing an effective amount of a T2-RNase or a polynucleotide encoding same may be directly injected into a target cancer or tumor tissue by a needle in amounts effective to treat the tumor cells of the target tissue.

For transmucosal administration, penetrants appropriate to the barrier to be permeated are used in the formulation. Such penetrants are generally known in the art.

For oral administration, the pharmaceutical composition of the present invention can be formulated readily by combining a T2-RNase or a polynucleotide encoding same with pharmaceutically acceptable carriers well known in the art. Such carriers enable a T2-RNase or a polynucleotide encoding same to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active ingredient doses.

Additional pharmaceutical compositions, which can be used orally, include push-fit capsules made of gelatin as well as soft, sealed capsules made of gelatin and a plasticizer, such as glycerol or sorbitol. The push-fit capsules may contain a T2-RNase or a polynucleotide encoding same in admixture with filler such as lactose, binders such as starches, lubricants such as talc or magnesium stearate and, optionally, stabilizers. In soft capsules, a T2-RNase or a polynucleotide encoding same may be dissolved or suspended in suitable liquids, such as fatty oils, liquid paraffin, or liquid polyethylene glycols. In addition, stabilizers may be added. All formulations for oral administration should be in dosages suitable for the chosen route of administration.

Oral delivery of the pharmaceutical composition of the present invention may not be successful due to the pH and enzyme degradation present in the gastrointestinal tract. Thus, such pharmaceutical compositions must be formulated to avoid undesirable circumstances. For example, enteric coating can be applied to oral solid formulation. Substances with acidic-resistant properties such as cellulose acetate phtalate (CAP), hydroxypropyl methycellulose phtalate (HPMCP) and acrylic resins are most commonly used for coating tablets or granules for micro encapsulation. Preferably wet granulation is used to prepare the enteric-coated granules to avoid reactions between the active ingredient and the coating (Lin, S.Y. and Kawashima, Y. 1987, Pharmaceutical Res. 4:70-74). A solvent evaporation method can also be used. The solvent evaporation method was used to encapsulate insulin administered to diabetic rats to maintain blood glucose concentration (Lin, S.Y. et al., 1986, Biomater, Medicine Device, Artificial organ 13:187-201 and Lin, S.Y. et al., 1988, Biochemical Artificial Cells Artificial Organ 16:815-828). It was also used to encapsulate biological materials of high molecular weight such as vial antigen and concanavalin A (Maharaj, I. Et al. 1984, J. Phamac. Sci. 73:39-42).

For buccal administration, the pharmaceutical composition of the present invention may take the form of tablets or lozenges formulated in conventional manner.

For rectal administration propositories can be used as is well known in the art.

For administration by inhalation, a T2-RNase or a polynucleotide encoding same for use according to the present invention is conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in an inhaler or insufflator may be formulated containing a powder mix of a T2-RNase or a polynucleotide encoding same and a suitable powder base such as lactose or starch.

The pharmaceutical composition of the present invention may also be formulated for parenteral administration, e.g., by bolus injection or continuos infusion. A composition for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of a T2-RNase or a polynucleotide encoding same may be prepared as appropriate oily injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of a T2-RNase or a polynucleotide encoding same to allow for the preparation of highly concentrated solutions.

Alternatively, a T2-RNase or a polynucleotide encoding same may be in a powder form for constitution with a suitable vehicle, e.g., sterile, pyrogen-free water, before use.

The pharmaceutical composition of the present invention may also be formulated in rectal compositions such as suppositories or retention enemas, using, e.g., conventional suppository bases such as cocoa butter or other glycerides.

In addition, a cancer or tumor present in a body cavity, such as in the eye, gastrointestinal tract, genitourinary tract (e.g., the urinary bladder), pulmonary and bronchial system and the like, can receive a physiologically appropriate composition (e.g., a solution such as a saline or phosphate buffer, a suspension, or an emulsion, which is sterile) containing an effective amount of a T2-RNase or a polynucleotide encoding same via direct injection with a needle or via a catheter or other delivery tube placed into the cancer or tumor afflicted hollow organ. Any effective imaging device such as X-ray, sonogram, or fiber optic visualization system may be used to locate the target tissue and guide the needle or catheter tube in proximity thereto.

The pharmaceutical composition of the present invention can also be delivered by osmotic micro pumps. The osmotic micro pumps are implanted into one of the body cavities and the drug is constantly released onto the tissue to be treated. This method is particularly advantageous when an immune response to the pharmaceutical composition is experienced. This method has been employed for ONCONASE (Vasandani V.M., et al., 1996, Cancer Res. 15;56(18):4180-6).

Alternatively and according to another preferred embodiment of the present invention, the pharmaceutically acceptable carrier includes a delivery vehicle capable of delivering a T2-RNase or a polynucleotide encoding same to the mammalian cell of the subject.

Numerous delivery vehicles and methods are known in the art for targeting proteins or nucleic acids into or onto tumors or cancer cells. For example, liposomes are artificial membrane vesicles that are available to deliver proteins or nucleic acids into target cells (Newton, A.C. and Huestis, W.H., Biochemistry, 1988, 27:4655-4659; Tanswell, A.K. et al., 1990, Biochmica et Biophysica Acta, 1044:269-274; and Ceccoll, J. et al., Journal of Investigative Dermatology, 1989, 93:190-194). Thus, a T2-RNase or a polynucleotide encoding same can be encapsulated at high efficiency with liposome vesicles and delivered into mammalian cells. In addition, the T2-RNase protein or nucleic acid can also be delivered to target tumor or cancer cells via micelles as described in, for example, U.S. Pat No. 5,925,628 to Lee, .

Liposome or micelle encapsulated T2-RNase or a polynucleotide encoding same may be administered topically, intraocularly, parenterally, intranasally, intratracheally, intrabronchially, intramuscularly, subcutaneously or by any other effective means at a dose efficacious to treat the abnormally proliferating cells of the target tissue. The liposomes may be administered in any physiologically appropriate composition containing an effective amount of encapsulated T2-RNase or a polynucleotide encoding same.

Alternatively and according to another preferred embodiment of the present invention the delivery vehicle can be, but it is not limited to, an antibody or a ligand capable of binding a specific cell surface receptor or marker. An antibody or ligand can be directly linked to a T2-RNase protein or nucleic acid via a suitable linker, or alternatively such an antibody or ligand can be provided on the surface of a liposome encapsulating a T2-RNase or a polynucleotide encoding same.

For example, a T2-RNase or a polynucleotide encoding same can be fused with specific membranal protein antibodies or ligands for targeting to specific tissues or cells as previously described in the art. It will be appreciated in this respect that fusion of RNase A of the ribonuclease A superfamily with antibodies to the transferrin receptor or to the T cell antigen CD5 lead to inhibition of protein synthesis in tumor cells carrying a specific receptor for each of the above toxins (Rybak, M. et al., 1991, J. Biol. Chem. 266:21202-21207 and Newton DL, et al., 1997, Protein Eng. 10(4):463-70).

Pharmaceutical compositions suitable for use in context of the present invention include compositions wherein the active ingredients are contained in an amount effective to achieve the intended purpose. More specifically, a therapeutically effective amount means an amount of the active ingredients effective to prevent, alleviate or ameliorate symptoms of disease or prolong the survival of the subject being treated.

Determination of a therapeutically effective amount is well within the capability of those skilled in the art, especially in light of the detailed disclosure provided herein.

Toxicity and therapeutic efficacy of the active ingredients described herein can be determined by standard pharmaceutical procedures in cell cultures or experimental animals, e.g., by determining the IC₅₀ and the LD₅₀ (lethal dose causing death in 50 % of the tested animals) for a subject active ingredient. The data obtained from these cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Depending on the severity and responsiveness of the condition to be treated, dosing can also be a single administration of a slow release composition, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

As has already been mentioned hereinabove, according to an aspect of the present invention, the active ingredient of the pharmaceutical composition is a polynucleotide encoding a T2-RNase.

According to this aspect of the present invention the polynucleotide is introduced into the mammalian cell along with a pharmaceutically acceptable carrier, which introduction results in a genetic modification of this cell, enabling the expression of a T2-RNase therein.

As used herein in the specification and in the claims section below, the term "genetic modification" refers to a process of inserting nucleic acids into cells. The insertion may, for example, be effected by viral infection, injection, transfection, particle bombardment or any other means effective in introducing nucleic acids into cells, some of which are further detailed hereinbelow. Following the genetic modification the nucleic acid is either integrated in all or part, to the cell's genome (DNA), or remains external to the cell's genome, thereby providing stably modified or transiently modified cells.

As such, the pharmaceutical composition according to this aspect of the present invention is usable for gene therapy.

As used herein the phrases "gene therapy" or "genetic therapy" are used interchangeably and refer to a method of therapy in which a stable or transient genetic modification of a proliferative cell(s) such as a cancer cell, leads to the inhibition of proliferation of this cell.

Any one of the polynucleotides identified in Table 2 by its Gene Bank accession number can be employed according to the present invention as a polynucleotide encoding a T2-RNase. In addition, polynucleotides 40 % or more homologous and/or hybridizing under mild and/or stringent hybridization conditions with the listed polynucleotides can also be employed as a polynucleotide encoding a T2-RNase, provided that the protein encoded thereby is characterized as a T2-RNase and exhibits the desired activities. Furthermore, it will be appreciated that portions, mutants chimeras or alleles of such polynucleotides can also be employed as a polynucleotide encoding a T2-RNase according to the present invention, again, provided that such portions, mutants chimeras or alleles of such polynucleotides encode a T2-RNase which exhibits the desired activities.

Isolation of novel polynucleotides encoding T2-RNases is also envisaged. Such isolation can be effected using methodologies well known in the art such as, but not limited to, library screening, hybridization, PCR amplification, labeled primers, labeled degenerated primers. Both genomic and cDNA polynucleotides can thus be employed.

A polynucleotide according to the present invention can be fused, in frame, to any other protein encoding polypeptide to encode for a fused protein using methods well known in the art. For example the polypeptide can be fused to a leader sequence or a signal peptide for secretion. Similarly a T2-RNase protein can be fused (conjugated) to other proteins using methods well known in the art. Many methods are known in the art to conjugate or fuse (couple) molecules of different types, including proteins. These methods can be used according to the present invention to couple a T2-RNase to other molecules such as ligands or antibodies to thereby assist in targeting and binding of the T2-RNase to specific cell types. Any pair of proteins can be conjugated or fused together using any conjugation method known to one skilled in the art. The proteins can be conjugated using a 3-(2-pyridyldithio)propionic acid Nhydroxysuccinimide ester (also called N-succinimidyl 3-(2pyridyldithio) propionate) ("SDPD") (Sigma, Cat. No. P-3415), a gluteraldehyde conjugation procedure or a carbodiimide conjugation procedure.

According to a preferred embodiment of the present invention, the polynucleotide includes one or more segments harboring transcription control sequences operatively linked to the T2-RNase encoding sequence. Such transcription control sequences can include, but are not limited to, promoters and enhancers as further detailed hereinbelow. These transcriptional control sequences are typically operatively linked upstream to the coding region and function in regulating the transcription and/or translation thereof.

According to another preferred embodiment of the present invention the polynucleotide encoding a T2-RNase is included within a eukaryotic expression vector. The phrase "expression vector" refers to a nucleic acid sequence which includes a sequence encoding a T2-RNase and transcriptional control sequences and which is capable of expressing a T2-RNase within a mammalian cell.

Numerous methods for the insertion of DNA fragments into a vector, for the purposes of mammalian gene expression are known in the art and may be used to construct a T2-RNase encoding gene expression vector including appropriate transcriptional/translational control sequences and the desired T2-RNase polynucleotide sequences. These methods may include *in vitro* DNA recombinant and synthetic techniques and *in vivo* genetic recombination. Expression of a polynucleotide encoding a T2-RNase may be regulated by transcription control sequences so that a T2-RNase is expressed in a host cell infected or transfected with the recombinant DNA molecule. For example, expression of a T2-RNase may be controlled by any promoter/enhancer element known in the art. The promoter activation may be tissue specific or inducible by a metabolic product or administered substance.

Promoters/enhancers which may be used to control T2-RNase expression within target tissues or cells include, but are not limited to, the native RB promoter, the cytomegalovirus (CMV) promoter/enhancer (Karasuyama, H., et al., 1989, J. Exp. Med., 169:13), the human β-actin promoter (Gunning, P., et al., 1987, Proc. Natl. Acad. Sci. USA, 84:4831-4835), the glucocorticoid-inducible promoter present in the mouse mammary tumor virus long terminal repeat (HHTV LTR) (Klessig, D. F., et al., 1984, Mol. Cell Biol., 4:1354-1362), the long terminal repeat sequences of Holoney murine leukemia virus (MULV LTR) (Weiss, R., et al., 1985, RNA Tumor Viruses, Cold Spring Harbor Laboratory, Cold Spring Harbor, New York), the SV40 early region promoter (Bernoist and Chambon, 1981, Nature 290:304-310), the promoter contained in the 3' long terminal repeat of Rous sarcoma virus (RSV) (Yamamoto et al., 1980, Cell 22:787-797), the herpes simplex virus (HSV) thymidine kinase promoter/enhancer (Wagner et al., 1981, Proc. Natl. Acad. Sci. U.S.A. 78:1441-1445), the regulatory sequences of the metallothionein gene (Brinster et al., 1982, Nature 296:39-42), the adenovirus promoter (Yamada et al., 1985, Proc. Natl. Acad. Sci. U.S.A. 82(11):3567-71), and the herpes simplex virus LAT promoter (Wolfe, J. H., et al., 1992, Nature Genetics, 1:379-384).

Expression vectors compatible with mammalian host cells for use in genetic therapy of tumor or cancer cells, include, but are not limited to, plasmids, retroviral vectors, adenovirus vectors, herpes viral vectors, and non-replicative avipox viruses, as disclosed, for example, by U.S. Pat. No. 5,174,993, .

Several methods can be used to deliver the expression vector according to this aspect of the present invention to the target mammalian cell(s).

For example, a suitable pharmaceutically acceptable carrier such as a physiologically appropriate solution, and which contains an effective amount of an expression vector can be administered topically, intraocularly, parenterally, orally, intranasally, intravenously, intramuscularly, subcutaneously or by any other effective means.

A physiologically appropriate solution containing an effective amount of an expression vector can be administered systemically into the blood circulation to treat a cancer or tumor which cannot be directly reached or anatomically isolated.

It will be appreciated that since a "naked" expression vector can be actively taken up by mammalian cell, uptake and targeted delivery is enhanced if the expression vector is appropriately packaged or encapsulated.

Thus, according to another preferred embodiment of the present invention the pharmaceutically acceptable carrier includes a delivery vehicle suitable for the delivery of the expression vector into mammalian cells in a targeted manner.

A viral expression vector may be introduced by a delivery vehicle into a target cell in an expressible form by infection or transduction. Such a delivery vehicle includes, but is not limited to, a retrovirus, an adenovirus, a herpes virus and an avipox virus. A delivery vehicle able to introduce the vector construct into a target cell and able to express T2-RNase therein in cell proliferation-inhibiting amounts can be administered by any effective method described hereinabove.

Alternatively, such a delivery vehicle can include, but is not limited to, a liposome, a micelle, an antibody or a ligand as previously described hereinabove.

It will be appreciated that the polynucleotides herein described can be used in the preparation of a medicament useful in inhibiting the proliferation of a mammalian cell of a mammal, by mixing the polynucleotide with an appropriate pharmaceutically acceptable carrier.

As already mentioned hereinabove, polynucleotides encoding a T2-RNase can be obtained by a variety of methods, including, but not limited to, polymerase chain reaction (PCR) amplification of genomic or cDNA libraries screening using T2-RNase specific primers, using reverse transcription PCR along with T2-RNase specific primers to amplify mRNA isolated from organisms known to express T2-RNases, or directly isolating DNA sequences coding for a T2-RNase from the appropriate organisms. It will be appreciated in this case that the above mentioned methods can also be used to isolate or generate any of the active forms of a T2-RNase described hereinabove.

The purified polynucleotide can then be inserted into appropriate expression vectors or provided with the appropriate transcriptional control sequences and prepared as described hereinabove.

As is further exemplified in the Examples section that follows and mentioned hereinabove, an assay for determining the effects of a specific T2-RNase or a polynucleotide encoding same is also provided in accordance with the teachings to the present invention. Such an assay is effected, for example, by exposing proliferating cells to a T2-RNase and following their proliferative behavior over time as compared to control, untreated cells. This assay can be employed not only for selecting for the most potent T2-RNase for any specific application, but also for establishing dose response, which can be translated into initial treatment dosage in *in vivo* experiments or during treatment of a subject, all as is further exemplified herein for RNase B1 of the T2 family. It will be appreciated that this assay can also be used to determine the anti-proliferative active site or portion or a T2-RNase, or to determine the activity of generated or isolated mutants which do not display ribonucleolytic activity.

Additional objects, advantages, and novel features of the present invention will become apparent to one ordinarily skilled in the art upon examination of the following examples, which are not intended to be limiting.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); . Other general references are provided throughout this document. The procedures therein are believed to be well known in the art and are provided for the convenience of the reader.

### EXAMPLE 1

### Characterization of Aspergillus niger B1 RNase and its inhibitory effect on pollen tube growth in fruit trees

### Materials and Methods

### Preparation and purification of A. niger extracellular RNase:

*Aspergillus niger* B1 (CMI CC 324626) was grown in liquid culture containing 1 % (w/v) wheat flour and 0.05 % (w/v) ammonium sulfate. The mixture was adjusted to pH 3.5 with hydrochloric acid and autoclaved. An inoculum of about 10⁶ spores was suspended in 100 ml of medium and incubated at 30 °C in an orbital shaker, at 200 rpm for 100 hours. The growth medium was passed through a 0.2-µm membrane and dialyzed three times against 10 volumes of 2 mM sodium acetate pH 6. Two liters of dialyzed solution were loaded onto a Fractogel EMD-TMAE 650 (M) 26/10 (Merck) column, equilibrated with 20 mM sodium acetate pH 6. Bound proteins were eluted with a 500-ml linear gradient of 0-1.0 M sodium chloride in the same buffer, using a fast protein liquid chromatography (FPLC) system (Pharmacia) with a flow rate of 5 ml·min⁻¹. The fractions exhibiting the highest RNase activity were pooled and dialyzed against 2 mM sodium acetate pH 6, and a 50-ml aliquot was loaded onto a MONO-Q 5/5 HR (Pharmacia) column, equilibrated with 20 mM sodium acetate pH 6. The elution was performed as with the EMD-TMAE column, except that only 10 ml of a 0-1.0 M salt gradient were used, at a flow rate of 1 ml·min⁻¹.

Proteins were monitored at 280 nm and measured according to Bradford (Bradford, M.M. 1976. Anal. Biochem. 72:248-245), using bovine serum albumin (BSA) as a standard. Different fractions were analyzed by a 12.5 % sodium dodecyl sulfate-polyacrylamide gel electrophoresis (SDS-PAGE) (Laemmeli, U.K. 1970. Nature 227: 680-685). RNase activity was determined as previously described (Roiz and Shoseyov1995, Int. J. Plant Sci. 156:37-41).

The purified RNase B1 was enzymatically deglycosylated according to the procedure described by Broothaerts et al. (Broothaerts, W.P. et al. 1991. Sex. Pant Reprod. 4:258-266). The enzyme was mixed with 0.5 % (w/v) SDS and 5 % (w/v) β-mercapthoethanol and heated at 100 °C for 5 minutes. Once cooled, the reaction mixture was diluted 2.5-fold with a buffer containing 50 mM sodium phosphate pH 7.5, 25 mM EDTA, 1 % (w/v) Triton X-100 and 0.02 % (w/v) sodium azide. Peptide-N-glycosidase F (PNGase F, Boehringer-Mannheim) was added to a final concentration of 20 units·ml⁻¹ and incubation was performed overnight at 37 °C. The sample was then mixed with sample application buffer, heated at 100 °C for 5 minutes, and analyzed by SDS-PAGE using a 12.5 % gel.

### RNase assays:

The optimal conditions for RNase activity were determined according to a procedure modified from Brown and Ho (Brown, P.H. and Ho, T.H.D. 1986 Plant Physiol. 82:801-806), using a range of temperatures from 20-100 °C at 10 °C increments, and a range of pH's from 2.5-7 at 0.5 pH units increments, established using 50 and 12 mM phosphate-citrate buffers. Samples of 10 µl were each added to 490 µl of ice-cold buffer, containing 4 mg·ml⁻¹ yeast RNA (Sigma). Half of each sample was used as a blank by immediately adding a stop solution containing 50 µl of 0.75 % (w/v) uranyl sulfate in 25 % (w/v) perchloric acid. The remaining half was incubated for 10 minutes, following which a 50 µl of stop solution was added to each. Following centrifugation at 15,000 X g for 5 minutes, the supernatant was diluted 20-fold with distilled water and the absorbance was determined at 260 nm. One unit of RNase activity was determined as the amount of enzyme releasing soluble nucleotides at a rate of one A.U_{260 nm} per min.

RNase B1 was visualized by an activity gel, as previously described (Roiz and Shoseyov, 1995, Int. J. Plant Scizzz. 156:37-41). An SDS gel containing RNase B1 was renatured by washing twice for 15 minutes each with 20 mM acetate buffer at pH 3.5 containing 25 % (v/v) iso-propanol and then twice for 15 minutes each with buffer alone. The renatured gel containing the RNase B-1 was laid over a plate containing 0.1 % RNA and 0.8 % agarose in 20 mM acetate buffer and incubated at 37 °C for 30 minutes. The gel was then removed and the agarose plate was stained with 0.02 % (w/v) toluidine blue in water to visualize RNase activity

### The effect of RNase B1 on pollen tube growth:

Peach cv. Almog pollen was germinated *in vitro* in liquid culture, as previously described (Roiz and Shoseyov, 1995, Int. J. Plant Sci. 156:37-41). Pollen grains were suspended in aliquots containing 100 µl of 15 % (w/v) sucrose, 100 µg·ml⁻¹ boric acid, 200 µg·ml⁻¹ magnesium sulfate, 200 µg·ml⁻¹ calcium nitrate and different concentrations of RNase B1. Following incubation overnight at 25 °C in a dark chamber, germination percentage was recorded. Pollen tube length was examined with an eyepiece micrometer.

The effect of RNase B1 treatment on pollen tube growth was also tested *in vivo.* Intact flowers of peach and in tangerine (*Citrus reticulata,* Blanco cv. Murcott) were sprayed at the early stages of anthesis with 100 units·ml⁻¹ RNase B1 in 20 mM citrate buffer at pH 3.5. In each species additional flowers at the same stage, on different branches, were sprayed with buffer alone or remained untreated as controls. Following exposure to open pollination for 48 hours, the styles were fixed in a 3:1 acetic acid to ethanol (by volume) for 24 hours, washed with distilled water and imbibed overnight in 8 M sodium hydroxide. Following thorough washing in distilled water, the styles were cut longitudinally, immersed each in a drop of 0. 1% (w/v) aniline blue in 0.1 M potassium phosphate on a slide and carefully squashed with a cover glass. Pollen tubes were observed by epifluorescence microscopy (Olympus BX40 equipped with WIB cube).

### The effect of RNase B1 on fruit set:

Field experiments were done in nectarine (*Prunus persica* var. Nectarina Fantasia). Branches of 30-40 cm long, bearing approximately 10 % open flowers, were sprayed with different concentrations of RNase B1 in 20 mM citrate buffer pH 3.5 and 0.025 % triton-X 100. Untreated branches, and branches sprayed with only buffer and triton-X 100, served as controls. The branches were sprayed at 2- to 3-days intervals during the blooming period (14 days). A month later, the number of fruit per branch was examined. For viability test, seeds were cut longitudinally through the embryo and immersed in 1 % 2,3,5-Triphenyl tetrazoluim chloride in water for 4 hours at 20 °C in a dark room. Red staining indicated viable tissues.

### Experimental Results

### Purification and characterization of RNase B1:

*A. niger* grown in liquid culture produced considerable amounts of extracellular RNase B1. A temperature of 60 °C and a pH of 3.5 were found optimal for RNase activity, and were adopted as the standard conditions for subsequent RNase assays.

RNase B1 purification included three steps (Table 3). In a first step a crude filtrate contained 1000 units·ml⁻¹ and 0.05 mg·ml⁻¹ protein was obtained. The crude filtrate was passed through an EMD-TMAE column and the pooled active fractions (Figure 1, graph A) contained 0.1 mg·ml⁻¹ protein, with an RNase activity of 40,000 units·ml⁻¹. In the final step, the pooled fractions were passed through a MONO-Q column and the active RNase fraction was eluted (Figure 1, graph B). This fraction contained a protein concentration of 1.05 µg·ml⁻¹ and RNase activity of 543,000 units·ml⁻¹. Two major protein bands, of 40 and 32 kDa, were observed following SDS-PAGE of the purified RNase B1 fraction (Figure 2). An RNase activity gel showed active bands corresponding to the 32 and the 40 kDa proteins. When subjected to PNGase F, a single protein band appeared at 29 kDa. RNase activity was retained after PNGase digestion (not shown).

**TABLE 3**

| Purification step | Total units | Protein concentration (mg/ml) | Recovery (%) | Specific activity (units/mg protein) |
|---|---|---|---|---|
| Crude filtrate | 0 | 0.05 | 100 | 20,000 |
| EMD-TMAE column | 0 | 0.1 | 56 | 400,000 |
| MONO-Q column | 652,200 | 1.05 | 32.6 | 517,143 |

### The effect of RNase B1 on pollen tubes and fruit set:

In *in vitro,* experiments 75 % of the control pollen grains germinated and the pollen tubes reached about 0.5 mm in length. Addition of RNase B1 to the growth medium reduced the percentage of germination and the length of the pollen tubes, in a dose responsive manner (Figure 3). RNase B1 had a pronounced inhibitory effect, 50 units·ml⁻¹, representing 0.1 µg·ml⁻¹ protein, were lethal, whereas 125 µg·ml⁻¹ of BSA reduced only half of pollen germinability and tube growth.

*In vivo,* control pollen tubes of peach were observed growing through the stigmatic tissue directed into the style 48 hours after pollination (Figure 4a). A similar effect was observed in styles treated with buffer only. In contrast, pollen grains germinated on stigmas treated with RNase B1 produced short pollen tubes, which appeared to lack any growth orientation, and failed to penetrate the stylar tissue (Figure 4b). In tangerine only a small portion of the stigmatic tissue, the diameter of which was 2-3 mm, was captured by the view field of the microscope. Therefore, only few pollen tubes were observed, as shown in Figure 5. However, the difference between the normal growth of the control pollen tubes (Figure 5a) and the irregular growth of the RNase-treated pollen tubes (Figure 5b), was clearly evident.

In nectarine cv. Fantasia, RNase B1 caused a reduction in fruit set (Table 4). In branches that remained untreated or sprayed with buffer with triton X-100, fruit set was 48.3 % and 36.3 %, respectively. It seemed that the low pH-buffer had some inhibitory effect on fruit set, however branches treated with 500 and 1000 units·ml⁻¹ of RNase B1 set 23.3 % and 18.4 % fruits, respectively, indicating a significant thinning effect of the RNase, in a dose dependent manner.

**TABLE 4**

| Treatment | Flowers (total number) | Fruit set (%) |
|---|---|---|
| Control untreated | 169 | 48.3 a* |
| Control buffer | 143 | 36.3 ab |
| 500 units/ml RNase B1 | 148 | 23.3 bc |
| 1000 units/ml RNase B1 | 106 | 18.4 c |

| | | |
|---|---|---|
| * values not sharing a common letter are significantly different at P = 0.05. | | |

In RNase B1 treated branches many undeveloped fruitlets were observed. Viability tests showed that in the control flowers (either untreated or sprayed with buffer only), embryo tissues were stained red, (Figure 6a), whereas the tissues of embryos developed in RNase-treated flowers, stained brown indicative of necrosis (Figure 6b).

*Aspergillus niger* B1 extracellular RNase (RNase B1) was purified to homogeneity. It was found to contain two isoforms of 32- and 40- kDa glycoproteins, sharing a 29-kDa protein core. The optimal RNase activity was observed at a temperature of 60 °C and a pH of 3.5. In peach (*Prunus persica* cv. Almog) and tangerine (*Citrus reticulata,* Blanco cv. Murcott) the enzyme inhibited pollen germination and tube growth *in vitro* as well as *in vivo.* In field experiments, the RNase caused a reduction in nectarine (*Prunus persica* var. nectarina Fantasia) fruit set and inhibited normal embryo development.

### EXAMPLE 2

### Inhibition of pollen germination and tube growth by T2-RNase is mediated through interaction with actin

The inhibition of pollen germination and tube growth by RNase is well recognized, yet the mechanism by which this enzyme interferes with the elongation process is still unclear. As such, this study set out to decipher the role of RNase B1 in interfering with the elongation process of pollen tubes.

### Materials and Experimental Methods

### The effect of RNase B1 on pollen tubes growth:

Anthers of lily (*Lilium grandiflorum* L. cv. Osnat) were let to dehisce for 24 hours at room temperature and than either used fresh or stored at -20 °C. RNase B1 was produced and purified from *Aspergillus niger* growth medium filtrate as described in Example 1. Pollen was germinated *in vitro* in aqueous cultures of 100 µl each, containing 7 % sucrose, 1.27 mM CaNO₃ 0.16 mM H₃BO₃, 1 mM K₂NO₃ and 3 mM KH₂PO₄ in water (Yokota and Shimmen 1994). Some cultures were supplemented with RNase B1 having 100 units/ml of RNase activity to a final protein concentration of 16 µg/ml. Additional cultures were supplemented with RNase that was previously boiled for 30 minutes which produced the loss of 50 % of activity, or with autoclaved RNase lacking any catalytic activity. Following 2 hours of incubation at 25 °C in the dark, pollen tube length was measured under the microscope eyepiece micrometer. The pollen tubes were stained with IKI (0.3 % I₂ and 1.5 % KI in water) to detect starch bodies.

Actively extending 1-hour pollen tubes were transferred to glass cells on the microscope stage. The pollen tubes growth pattern and organelle movement were video recorded as modified from Heslop-Harrison and Heslop-Harrison (Heslop-Harrison, J. and Heslop-Harrison, Y. 1990. Sex Plant Reprod. 3:187-194), using Applitec MSV-800 video presenter. Images were captured at 0.8 frames/sec for 8 seconds by Scion LG-3 frame grabber and then digitized and integrated by NIH *image* software. The photographs were processed using Adobe Photoshop (Adobe Systems Inc., Mountain View, CA) and Power-Point (Microsoft Co.) softwares.

### The effect of RNase on pollen tube actin filaments:

Pollen was germinated *in vitro* in aqueous cultures with or without RNase. Following incubation overnight, the pollen tubes were gently pelleted and the growth medium was replaced with 10⁻⁶ M tetramethylrhodamine B isothiocyanate (TRITC)-labeled phalloidin (Sigma) in PBST buffer (150 mM NaCl, 3 mM KCl, 10 mM Na₂HPO₄, 2 mM KH₂PO₄ and 0.02 % Tween-20). For *in vivo* observations, lily cv. Stargazer flowers were emasculated at the onset of anthesis and 0.5 ml of a growth medium containing 100 units/ml RNase were injected through the stigma into the stylar canal. Flowers into which growth medium without RNase was injected were used as a control. The liquids were absorbed into the stylar tissue for 5 hours at 25 °C, following which the stigmas were hand-pollinated by lily cv. Osnat pollen. Following 48 hours of incubation at 25 °C, each pistil was cut longitudinally and the pollen tubes were carefully excised and removed into TRITC-TBST solution and incubated for 1 hour. The incision at the stigma did not affect the pollen tubes, since their vital protoplasts were located at the distal part, protected by callose plugs. In both the *in vitro* and *in vivo* experiments the stained pollen tubes were rinsed in TBS (TBST lacking Tween-20), placed on a glass slide and observed with an epifluorescent light microscope (Olympus BX40 equipped with USH-102D mercury lamp).

### Binding of actin to RNase B1:

The interaction between RNase B1 and actin was quantified as modified from Simm (Simm, F.C. et al., 1987. Eur. J. Biochem. 166:49-54). Rabbit muscle globu(G-) actin (Sigma Co.) was polymerized to filamentous (F-) actin in Buffer F (10 mM Tris pH 8, 0.1 mM ATP, 0.2 mM CaCl₂, 0.1 M KCL and 2 mM MgCl₂) for 30 minutes at room temperature. Samples of 50 µl containing each 30 µM F-actin were incubated overnight at 4 °C with 1-33 µM RNase B1. As a control, each concentration of RNase was incubated with buffer F alone. The samples were centrifuged at 15,000 g for 40 minutes and RNase activity of the supernatant was determined (Roiz, L., Goren, R. and Shoseyov, O. 1995, Physiol. Plant. 94:585-590.).

### Immunogold silver staining of RNase B1 on pollen tubes:

Immunogold silver stain (IGSS) was used to detect RNase attachment to lily pollen tubes. Polyclonal antibodies were raised against RNase B1 in rabbit (Aminolab). *In vitro* 2-hours lily pollen tubes were fixed overnight in 2.5 % gluteraldehyde in PBST at 4 °C. The pollen tubes were washed for 1 hour in PBST, blocked for 1 hour in PBST containing 1 % BSA and 2 % skim-milk and incubated for 1 hour in anti-RNase B1, diluted 1:500 in PBST. Rabbit pre-immune serum (PIS) was used as control. The pollen tubes were washed three times, 10 minutes each, in PBST and then incubated for 1 hour in goat anti-rabbit IgG conjugated with 5-nm gold particles, diluted 1:100 in PBST. Following two 10 minutes-washes in PBST and one 10 minutes-wash in water, a silver-stain kit (BioCell Research Laboratories) was used for the final development of the reaction. The pollen tubes were soaked in the combined kit solutions for 10-15 minutes, washed in excess distilled water and observed under a light microscope (Olympus BX40).

### Experimental Results

A control sample of lily pollen tubes, germinated *in vitro* in a growth medium without RNase, reached about 300 µm in length (Figure 7). Cultures that were treated with RNase under the same conditions, reached only 160 µm in length. Boiling or autoclaving the RNase yielded pollen tubes of 130 and 170 µm in length, respectively. The differences between the three RNase-treated groups of pollen tubes were deemed insignificant.

Starch-staining showed that amylioplasts of the control sample were observed spreading along the pollen tube, except at the tip zone (Figure 8a). On the other hand, in RNase-treated pollen tubes IKI-stained bodies accumulated at the tip zone (Figure 8b).
The integrated video images of actively extending pollen tubes displayed the cytoplasmic flow lines (Figures 9a and 9b). In the control sample a continuous longitudinal movement was most common, as was acropetal flow at the tube periphery and basipetal flow at the center, forming an "inverse fountain" pattern beneath the tip zone (Figure 9a). The tip zone itself was occupied by much smaller bodies, mainly P-particles, the movement pattern of which was hardly observed. In RNase-inhibited pollen tubes the tip appeared swollen, with well-visible starch and lipid particles reaching the tip zone (Figure 9b). No continuous movement could be detected, but instead extended irregular images indicated cytoplasmic bodies rotating randomly.

The effect of RNase on the actin filaments distribution was examined in 1-hour *in vitro* and 48-hours *in vivo* pollen tubes. *The in vivo* pollen tubes reached about 3-4 cm long, and their TRITC-phalloidin staining was more intensive than in the *in vitro* tubes. However, The mode of the RNase effect was similar in both experiments. In the control, actin microfilaments were assembled longitudinally along the tube axis, forming a fine network in the tip zone (Figure 10a). On the other hand, in RNase-treated pollen tubes masses of actin were accumulated at the tip cell wall (Figure 10b).

The interaction between RNase B1 and actin was quantified using Scatchard analysis. In the actin-RNase B1 experiment a regression line, intersecting with the abscissa at 0.45 (Figure 11) indicated that the RNase:actin molar ratio was 0.45, implying that two actin molecules bind to each RNase molecule.

Pollen germinated in the presence of RNase B1 were prepared for light microscopy and the location of RNase was determined by IGSS, using anti-RNase antibodies (Figures 12a-c). In pollen tubes grown without RNase (Figure 12a) or with RNase but treated with PIS (Figure 12b), the cell wall external surface was devoid of silver staining. On the other hand, in pollen tube treated with RNase B1, a clear immunogold silver stain appeared, accumulating upon the tip zone (Figure 12c).

In this study Lily (*Lilium grandiflorum*) pollen germination and tube elongation were specifically inhibited by *A. niger* RNase B1. Boiled or autoclaved RNase, lacking most of the original catalytic activity, showed a similar inhibitory effect. The results demonstrate that *A. niger* RNase is an actin-binding protein having an inhibitory effect on pollen tube elongation. This binding which is unrelated to the catalytic activity of RNase B1 deforms the pollen tube actin filaments arrangement to thereby disrupt cytoplasmic streaming.

### EXAMPLE 3 (REFERENCE EXAMPLE)

### The effect of RNase B1 on human colon cancer cells

Since the actin binding activity uncovered for RNase B-1 in pollen tubes hinted at a possible cytotoxic activity it was decided to examine the cytotoxic effect of RNase B1 on human colon cancer cells.

### Materials and Experimental Methods and Results

### Cell culture:

All the experiments were performed *in vitro.* Human colon adenocarcinoma (HT29) cells were grown in DMEM medium (Biological Industries, Bet Haemek), supplemented with 10 % fetal calf serum, 1 % glutamine and 10 % Antibiotic-Antimicotic solution (Biolab). The cells were incubated at 37 °C in a humidified atmosphere containing 5 % CO₂. RNase B1 solutions were made in PBS buffer, pH 6.8.

### Preliminary cell viability assay:

Cells were incubated with 50-ml flasks. Each flask contained 2X10⁵ cells in 7 ml medium, in the absence or presence of different concentrations (10⁻⁸ - 10⁻⁶ M) of RNase B1. The cells were grown for 48 hours or 72 hours, and then viable and non-viable cells were differentially counted using trypan blue staining.

In all treatments the total number of cells grown for 72 hours (55-60 X 10⁵) exceeded by about twofold the number of cells obtained after 48 hours culture (25-30 X 10⁵) (Figure 13a). The presence of RNase B1 in the growth medium did not have a significant effect on cell growth. However, a small but significance effect of the RNase B1 on the number of dead cells was found at both 48 hours and 72 hours of incubation (Figure 13b).

### Clonogenicity assay I:

The long-term survival of tumor cells is characterized by their ability to divide and produce clones. Cells were preincubated for 48 hours in growth medium containing 10⁻⁶ M RNase B1, then trypsinized, washed and resuspended in growth medium lacking RNase B1. Prior to plating into 96-well microtiter plates, the cells were diluted to serial 5-fold dilutions ranging between 50-to-10⁵ cells in each well (200 ml). The plates were incubated for 14 days in the conditions described above, without adding fresh growth medium following which the colonies were fixed and stained with methylene blue. The clonogenic cells in each well were numbered following clones visualization. Control cells were treated as above, but preincubated during the first 48 hours in medium lacking RNase B1.

In both treatments a similar number of colonies was observed in wells in which 100 cells were plated (Figure 14). The cytotoxic effect of the RNase B1 appeared in wells containing higher densities of cells. In wells plated with 500 cells each, the control and the RNase B1-treated cells produced 180 and 100 colonies per well, respectively. Furthermore, in wells plated with 1000 cells each the RNase B1-treated cells formed about 250 colonies per well, whereas the control cells formed numerous colonies that fused to a continuous layer, thus could not be counted and illustrated in Figure 14. Cells plated at higher densities did not survive the culture without changing media.

### Clonogenicity assay II :

The ability of the tumor cells to proliferate and to colonize was examined in short vs. continuous exposure to RNase B1. The experiment was performed as described in Clonogenicity assay I using (i) control cells, (ii) cells preincubated with medium containing 10⁻⁶ M RNase B1 and then allowed to colonize in RNase B1-free growth medium, and (iii) cells preincubated as in (ii) and then incubated during the colonization assay in growth medium containing 10⁻⁶ M RNase B1. In these experiments the initial densities ranged between 250-1000 cells per well and the colonization period was 7 days.

The shorter period of incubation used in this experiment (7 days) compared to a 14 day preincubation resulted in non defused colonies, which could be distinguished even in wells containing high densities of cells. In all densities, 48 hours of preincubation in RNase B1 led to a reduction of 20-30 % in the ability of the cells to colonize, compared to the control (Figure 15). However in each density a continuous exposure to RNase B1 led to a dramatic reduction of 90 % in clonogenicity. Figures 16a-c Show that the continuous RNase B1-treated cells (Figure 16c) were smaller and less stainable than the cells that were preincubated for 48 hours in RNase B1 (Figure 16b) or the control cells (Figure 16a). This result indicate that RNase B1 affected the colonies growth rate.

Thus as is clearly shown from the results presented herein *A. niger* RNase B1 has a clear cytotoxic effect on human adenocarcinoma HT29 cancer cells. The cytotoxic effect of RNase B1 is expressed via reduction of cell clonogenicity, rather than reduction of cell viability. It is possible that the RNase B1 has a long-term effect on the tumor cells. The RNase B1 causes a reduction in the colonies growth rate compared to the control, indicating that it may affect the ability of the cells to proliferate.

### EXAMPLE 4 (REFERENCE EXAMPLE)

### The in vivo effect of RNase B1 on tumor development in a rat model

For further studying the anti-cancer effect of RNase B1, an *in vivo* experiment was conducted in rats.

### Materials and Experimental Methods

Charles-River derived 4-weeks old male rats were divided into groups of 6. In some groups, the rats were induced to develop colon cancer by five weekly injections of Dimethylhydrazine (DMH). In this experiment, two modes of RNase B1 administartion were examined. RNase B1 was applied directly into the colon by osmotic micro-pumps, or given orally using enterocoated microcapsules. The full set of treatments each rats group received is described by the scheme of Figure 1. During the experiment, the rats were weighted weekly to monitor the effect of DMH and/or RNase B1 on their growth rate. In groups treated with RNase B1 feces was collected weekly from each cage and dried at 60°C. A sample of 250 mg dry feces was grounded and re-dissolved in phosphate buffer saline (PBS). Following centrifugation, RNase activity in the upper solution was examined as described in Roiz *et al.* (Roiz, L. et al., J. Amer. Soc. Hort. Sci. 125(1):9-14.2000).

### Administration of RNase B1 to the colon via osmotic micro-pumps:

RNase B1 was loaded into osmotic micro-pumps (ALZET). The pumps were implanted subcoutanously into the rats abdomen. The osmotic pumps allowed a constant release of RNase B1 directly into the colon via a catheter, at a calculated concentration of 10⁻⁶ M in the colon for at least 6 weeks, assuming that the rat colon is about 4 ml in volume. Rats were treated as follows: Rats of one group were implanted with pumps containing "live" RNase B1 (RNase B1), having full RNase activity. Rats of a second group were implanted with pumps containing autoclave-inactivated RNase B1 (I-RNase B1), lacking any RNase activity. Whereas, rats of a third group were implanted with pumps containing PBS, which was used as the RNase B1 vehicle in the first two groups, and served as controls.

To examine a possible preventive effect of RNase B1, selected DMH-treated rats received RNase B1 1-9 weeks after the first DMH injection. Then, the rats were sacrificed and their colons excised and washed with PBS and then with PBS containing 0.1 M dithiothreitol (DTT). The colons were thereafter opened longitudinally and fixed for at least 1 hour in 4 % formaldehyde in PBS over a filter paper. Following staining with 0.025 % methylene blue in PBS, the colon mucosa was observed via a microscope under low magnification for aberrant crypt foci (ACF). ACF were counted in the distal (5 cm) colon.

To examine a therapeutic effect of RNase B1, the rest of the rats received RNase B1 from 12 to 17 weeks after first DMH injection. The colons were excised and fixed as described above and the tumors counted and measured. For histopathological examinations, each tumor was embedded in paraffin. Thin (10 µm) sections were stained and the degree of malignancy was evaluated.

### Oral administration of RNase B1:

### Preparation of microcapsules:

Microcapsules were prepared with a modified procedure described by Lin et al. (Lin J.J., et al., 1994, Biochem. Biophys. Res. Commun. 14; 204(1):156-62). A mixture of 0.6 grams lyophilized RNase B1 and 2.4 grams glucose was well powdered by a mortar and pestle. The fine powder was poured into a 1 L beaker containing 200 ml liquid paraffin and 2 ml Span-80 and stirred at 600 rpm for 20 min. Acetone-ethanol-cellulose acetate phthalate (CAP) solution was carefully added to the above stirring mixture (3.2 grams CAP in 40 ml of 9:1 acetone: 95 % ethanol) and let stir for further 2 h in a hood, to remove traces of acetone. The microcapsules were hardened by adding 30 ml ether and dried on filter paper using Buchner funnel and traces of liquid paraffin were removed by two additional washes with 30 ml ether. The microcapsules were then let dry overnight and pass through a fine mesh. Most microcapsules were between 200 and 500 µm.

In a preliminary experiment (Figure 18), CAP microcapsules were found insoluble in acidic pH, representing the stomach environment. However, after 1 hour in alkaline pH maximum RNase activity was reached, indicating that the microcapsules could easily release their content in the intestines.

### Oral administration of the RNase B1 microcapsules:

The microcapsules containing RNase B1 or glucose as placebo, were mixes with ground Purina chows. Each RNase B1-treated rat received a daily dose of 1.6 mg RNase B1, to gain a final concentration of 10⁻⁵ M in the colon. The experimental details for oral administartion were as described above for the micro-pumps administartion, except for a delayed termination (11 weeks) while evaluating the preventive effect of RNase B (Figure 17).

### Experimental Results

### The effect of the different treatments on rat growth rate:

The rats initial weight was about 200 grams. The experiment ended at different time for each treatment, as described above. Generally, the rats reached a final body weight of about 400-500 gramss, with no significant differences between the different treatments (Figure 19a-d). However, DMH-treated groups showed a slight decrease in body weight comparing to RNase B1-treated groups, in the presence or absence of DMH.

### RNase activity in rat feces:

Figures 20a-c show changes in RNase activity in feces of rats implanted with osmotic pump containing RNase B1, I-RNase B1 or PBS in a preventive mode (Figure 1) during 8 weeks. In rats treated with RNase B1 (Figure 20a) RNase activity in feces was 5-fold higher than in rats treated with I-RNase B1 (Figure 20b) or with PBS (Figure 20c). This activity was maintained high for 5 weeks and then decreased gradually, as the reservoir of RNase B1 in the pumps exhausted. A basal endogenous RNase activity was detected in feces of the two latter groups.

Similar pattern was observed in rats fed with microencapsulated RNase B1, but an 8-fold higher RNase activity was detected as is compared to rats fed with microcapsules containing glucose only (Figure 21). In this experiment, the gradually decrease in RNase activity may be explained as a result of increase in the rats body weight and as a consequence, the colonic volume.

### The effect of RNase B1 as a preventive agent:

The pump-implanted rats were sacrificed after 8 weeks, since infection at the pump sites was observed. At this stage only ACF were apparent. ACF are surrogated biomarkers of carcinogenic changes in the rat colon during the initiation phase of carcinogenesis. Goblet cells in cryptae become larger and were intensively stained comparing to the normal mucosal cells. ACF counts were dramatically reduced due to RNase B1 or I-RNase B1 treatments (Figure 22). No damaging effect on colon mucosa was observed in rats treated with RNase B1 or I-RNase B1 in the absence of DMH.

In rats fed with microencapsulated RNase B1, the experiment continued for 11 weeks following the first DMH administration. At that time, both tumors and ACF were present. RNase B1 caused a reduction in the number of tumors per colon (Figure 23a), in tumor size (Figure 23b) as well as in the number of ACFs (Figure 23c) as is compared to control.

In addition, a color diversity of colon tumors was observed; red tumors that had intensive blood supply (Figure 24a), white tumors almost devoid of blood vessels (Figure 24b), and "pink" tumors with only few blood vessels (Figure 24c). In glucose-treated rats all the tumors were red. On the other hand, in RNase B1-treated rats a significant reduction in the number of reddish tumors was observed (Figure 24d); 10 % and 50 % of the tumors were pink and white, respectively. These results clearly indicate an anti-angiogenic effect of RNase B1.

Tumors could also be distinguished by histopathological parameters, as benign or malignant. A benign tumor termed adenoma (Figure 25a) can be defined by a propagated mucosal layer, and sometimes by development of adenopapilloma, however the submucosa is intact and well definefrom the other colon layers. In a malignant tumor, termed adenocacinoma, mucosal cells penetrate beneath the submucosa and eventually lead to a loss of tissue arrangement (Figures 25b and 25c). Examinations of the distribution of the different types of tumors in rats treated preventively with glucose or RNase B1 showed that RNase B1 clearly reduced the degree of malignancy (Figure 9d).

### The effect of RNase B1 as a therapeutic agent:

In both modes of application, either directly by osmotic pumps or orally, the well-developed tumors were exposed to RNase B1 during weeks 12-17 of the experiment. In rats treated by osmotic pumps, RNase B1 caused a reduction in the number of tumors per colon (Figure 26a). The inhibitory effect, about 50 % relative to the control, was most significant in rats treated with I-RNase B1.

RNase B1 affected also tumor growth, as demonstrated by size distribution (Figure 26b). In general, most tumors had a diameter of 3-5 mm, however in PBS-treated rats exceptionally large tumors, of more than 9-12 mm, appeared. This result implies that RNase B1 inhibits or arrests the development of pre-existing tumors. Nonetheless, no significant differences between the effects of RNase B1 and I-RNase B1 were observed.

As in the experiment of RNase B1 preventive effect, the pattern of angiogenesis was also affected by RNase B1 applied via osmotic pumps (Figure 26c). In PBS-treated rats most of the tumors, about 80 %, were red in color. In contrast, in both RNase B1 and I-RNase B1 treated rats only 30 % of the tumors were red, whereas the other were pink or white. It appears that RNase B1 reduces angiogenesis also in pre-existing tumors.

In rats fed with encapsulated RNase B1, the effect of the treatment was less significant than that obtained by osmotic pumps (Figures 27a-c). This result is explained by assuming that a very small proportion of the protein reaches to the colon. As mentioned before, the microcapsules indeed pass the stomach, but they still have a long route through the small intestine and the cecum. An experiment to test this hypothesis was therefore conducted using CAP microcapsules loaded with a fluorescent protein and given to rats. The rats were sacrificed after 6 hours and the content of their gastrointestinal tract was observed under a fluorescent microscope. It was found that in the duodenum the microcapsules started to dissolve. The dissolution further processed in the ileum and jejunum. When the microcapsules reached into the cecum, most of the fluorescence was diffused into the cecum content. Since the microcapsules were damaged, RNase B1 effect may be reduced due to proteases present in the intestine and cecum.

Despite the fact that orally administered RNase B1 did not decrease the number and size of pre-existing tumors, the distribution among tumor types of color was slightly affected (Figure 27c). Glucose- and RNase B1 treated rats had about 60 % and 40 % red tumors, respectively. It appears that orally administrated RNase B1 in the present formulation also affect angiogenesis, but in a moderate way.

### EXAMPLE 5 (REFERENCE EXAMPLE)

### The effect of RNase B1 on human HT-29 colon cancer cells in vitro

### Material and Experimental Methods

### Cell growth conditions:

All experiments were performed in human colon adenocarcinoma (HT-29) cells. The cells were grown in 50-ml flasks containing DMEM medium (Biological Industries, Bet Haemek), supplemented with 10 % fetal calf serum, 1 % glutamine and 1 % Antibiotic-Antimicotic solution (Biolab). The cells were trypsinizated, and 2 ml medium containing 5x10⁴ cells were plated in each well of a 6-wells plate. Some plates were supplemented with RNase B1, to a final concentration of 10⁻⁶ M. The cells were incubated at 37°C in a humidified atmosphere containing 5 % CO₂. After 48 hours the medium in the presence or absence of RNase B1 was replaced in each well respectively, to maintain a constant supply of ingredients and RNase B1. After four days the medium was removed and the cell cultures were fixed in 4 % formaldehyde in PBS (150 mM NaCl, 3 mM KCl, 10 mM Na₂HPO₄, 2 mM NaH₂PO₄) pH 7.2 for 15 min on ice. The cells were stained aiming different purpose, as follows.

### Direct staining for intracellular actin:

The cells were washed in PBS and permeabilized in PBS containing 0.02 % Tween-20 (PBST) for 1 hour in room temperature. Following 3 washes with PBS, the cells were stained for actin with 10⁻⁶ M tetramethylrhodamine B isothiocyanate (TRITC)-labeled phalloidin (Sigma) for 1 hour and let stand in PBS overnight at 4°C to remove any excess of staining material. The cells were spread in water on a glass slide and visualized using Confocal Laser Scanning Microscope (LSM) 510 (Zeiss).

### Immunostaining for membranal actin:

The cells were fixed with formaldehyde and washed with PBS as described above, and then incubated with rabit anti-actin antibodies (Sigma) diluted 1:500 in PBS for 1 hour at room temperature and washed three times with PBS. The cells were then incubated with goat anti-rabbit IgG conjugated to fluorescein isothiocyanate (FITC) diluted 1:100 in PBS for another 1 hour at the same conditions, washed again and visualized as described above.

### Immunostaining of RNase B1 on cell surface:

Polyclonal antibodies (Aminolab, Israel) were raised in rabbit against purified RNase B1. Anti-RNase B 1 was used as primary antibody in HT-29 immunostaining, according to the procedure describe above.

### Experimental Results

### Direct staining for intracellular actin:

In control cells growing without RNase B1, a fine actin network was observed stained with TRITC, filling the cell cytoplasm. A light stain was observed at the membrane surface (Figure 28a). In contrast, in RNase B1-treated cells the membrane and the peripheral zone of the cytoplasm in each cell was intensively stained (Figure 28b), indicating rearrangement of actin network as a response to external addition of RNase B1.

### Immunostaining for membranal actin:

The FITC-immunostaining showed fine fluorescent spots of actin at the membranal zone in control cells (Figure 29a). This result coincides with the TRITC staining shown in Figure 28a. In this experiment, no detergent was used, thus the antibody hardly penetrated into the cells cytoplasm. Therefore, it appears that in these cells, membranal actin interacts with the external environment. In RNase B1-treated cells a much weaker immunostaining was observed (Figure 29b), implying that RNase B 1 previously bound to membranal actin interfered with the binding of the anti-actin antibodies.

Additional cells, not treated with RNase B1, were incubated with the previously mixed rabbit anti-actin and 1 µM actin. A similar faint fluorescence was observed, as described in Figure 29b (not shown). To eliminate the possibility of spontaneous FITC fluorescence, cells were treated as describe, except anti-actin was omitted. No fluorescent staining was detected.

### Immunostaining of RNase B1 on cell surface:

Very faint FITC-fluorescence appeared in control cells incubated with anti-RNase B1 (Figure 30a). However, RNase B1-treated cells exhibited an intense fluorescent response (Figure 30b). This result indicates a significant presence of RNase B 1 over the cell surface, especially on the cells edges and extensions. Treatment with rabbit pre-immune serum (PIS) instead of anti RNase B 1 resulted in a very faint fluorescence (Figure 30c).

### EXAMPLE 6

### The effect of IAc-RNase B1 on lily pollen tubes growth

### Materials and Experimental Methods

### Iodoacetylation of RNase B1:

Iodoacetylation of RNase B 1 was performed according to Irie et al (Irie M, et al. 1986 J. Biochem. 99(3):627-33). RNase B 1 was dissolved in 2.5 ml of 0.1 M acetate buffer, containing 0.1 M iodoacetate, to a final concentration of 10 nM. Following incubation overnight at 37°C, the protein was desalted on a Sephadex G-15 column. The fractions containing the protein were collected and intensively dialyzed against water. After lyophilization, 10 mg of protein were obtained. The iodoacetylated (IAc-) RNase B 1 RNase activity was compared to the non-modified RNase B 1.

### The effect of IAc-RNase B1 on lily pollen tubes:

Lyophilized IAc-RNase B 1 was dissolved to a final concentration of 1 or 5 µM in lily pollen tube growth medium containing 7 % sucrose, 1.27 mM Ca(NO₃)₂, 0.16 mM H₃BO₃, 1 mM KNO₃ and 3 mM KH₂PO₄ in water (Yokota, E. and Shimmen, T. 1994. Protoplasma 177: 153-162). Lily (*Lilium longiflorum*) pollen grains were germinated *in vitro* in tubes containing 100 µl of growth medium, in the presence or absence of 10⁻⁶ M IAc-RNase B1. As additional controls, lily pollen was germinated in the same concentrations of RNase B1 or BSA in growth medium. Following 1.5 hour incubation at 25°C in darkness, pollen tube length was measured in each treatment under light microscope.

### Experimental Results

### Iodoacetylation of RNase B1:

Iodoacetate leads to the inhibition of RNase activity, via binding to histidine residues in the active site of RNase. RNase activity of IAc-RNase B 1 was 90 % less, compared with non-treated RNase B 1.

### The effect of IAc-RNase B1 on lily pollen tubes growth:

Control lily pollen tubes reach a length of 0.26 mm (Figure 31). In this experiment, BSA was used as a control, since it does not have any cytotoxic effect on pollen tubes. Indeed, at a concentration of 10⁻⁶ M, BSA did not have a significant effect on pollen tube growth. The inhibitory effect of 5x10⁻⁶ M BSA may be explained as a result of the fact that pollen tubes are sensitive to changes in the growth medium osmotic potential. Both RNase B1 and IAc-RNase B1 exhibited a clear inhibitory effect on pollen tube growth. In both concentrations IAc-RNase B 1 was more effective than RNase B1, however the differences were not significant. Thus, in lily pollen tubes, IAc-RNase B1 inhibits pollen tubes growth in a similar manner of non-modified RNase B1, showing that loss of RNase activity does not reduce its inhibitory effect.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents, patent applications and sequences identified by an accession number mentioned in this specification are herein incorporated in their entirety by reference into the specification, to the same extent as if each individual publication, patent, patent application or sequence was specifically and individually indicated to be incorporated herein by reference. In addition, citation or identification of any reference in this application shall not be construed as an admission that such reference is available as prior art to the present invention.

## Claims

1. A ribonuclease of the T2 family for use in inhibiting tumor angiogenesis in a subject, wherein the ribonuclease of the T2 family binds actin in either its active or non-active ribonucleolytic form, wherein said ribonuclease is **characterized by** a molecular weight of the T2 protein of 24 to 36 KDa and acid pH optimum for RNase activity.

2. A polynucleotide encoding and capable of expressing in vivo a recombinant ribonuclease of the T2 family for use in inhibiting tumor angiogenesis in a subject, wherein the ribonuclease of the T2 family binds actin in either its active or non-active ribonucleolytic form.

3. The ribonuclease of claim 1 or polynucleotide of claim 2, wherein said tumor angiogenesis is associated with abnormally proliferating cells associated with a proliferative disorder or disease selected from the group consisting of papilloma, blastoglioma, Kaposi's sarcoma, melanoma, lung cancer, ovarian cancer, prostate cancer, squamous cell carcinoma, astrocytoma, head cancer, neck cancer, bladder cancer, breast cancer, lung cancer, colorectal cancer, thyroid cancer, pancreatic cancer, gastric cancer, hepatocellular carcinoma, leukemia, lymphoma, Hodgkin's disease, Burkitt's disease.

4. The ribonuclease of claim 1 or polynucleotide of claim 2 or 3, wherein said ribonuclease of the T2 family is selected from the group consisting of RNase HI0526, RNase I, RNase T2, RNase Rh, RNase M, RNase Trv, RNase Irp, RNase Irp1, RNase Le2, RNase Phyb, RNS2, RNase 3, RNase 1, RNase LE, RNase LX, S-RNase, RNase MC, RNaseCL1, RNase Bsp1, RNase RCL2, RNase Dm, RNase Oy, RNase Tp and RNase 6P1.

5. The ribonuclease of claim 1 or polynucleotide of claim 2, wherein said tumor angiogenesis is associated with a malignant tumor.

6. The ribonuclease of claim 1 or 5 or polynucleotide of claim 2, wherein said tumor angiogenesis is associated with a primary tumor.

7. The ribonuclease of claim 1 or 6 or polynucleotide of claim 2 wherein said ribonuclease of the T2 family is *A. niger* T2 RNase.

8. The ribonuclease of claim 1 or 7 or polynucleotide of claim 2, wherein said ribonuclease of the T2 family is devoid of ribonuclease activity.

9. The ribonuclease of claim 1 or 8 or polynucleotide of claim 2, wherein an actin binding activity of said ribonuclease protein is boiling stable.

10. The ribonuclease of claim 1 or 9 or polynucleotide of claim 2 wherein said tumor angiogenesis is angiogenesis of a metastatic tumor.

11. A ribonuclease of the T2 family for use in treating a condition associated with abnormal cell proliferation in a subject, wherein the ribonuclease of the T2 family binds actin in either its active or non-active ribonucleolytic form, wherein said ribonuclease is **characterized by** a molecular weight of the T2 protein of 24 to 36 kDa and acid pH optimum for RNase activity, and wherein said condition is selected from the group consisting of arthritis, rheumatoid arthritis, diabetic retinopathy, restenosis, in-stent restenosis and vascular graft restenosis.

12. A polynucleotide encoding and capable of expressing in vivo a recombinant ribonuclease of the T2 family for use in treating a condition associated with abnormal cell proliferation in a subject, wherein the ribonuclease of the T2 family binds actin in either its active or non-active ribonucleolytic form, wherein said ribonuclease is **characterized by** a molecular weight of the T2 protein of 24 to 36 kDa and acid pH optimum for RNase activity, and wherein said condition is selected from the group consisting of arthritis, rheumatoid arthritis, diabetic retinopathy, restenosis, in-stent restenosis and vascular graft restenosis.

## Patentansprüche

1. Ribonuklease der T2-Familie zur Verwendung bei der Hemmung der Tumor-Angiogenese in einer Testperson, wobei die Ribonuklease der T2-Familie Actin entweder in seiner aktiven oder nicht aktiven ribonukleolytischer Form bindet, wobei die genannte Ribonuklease durch ein Molekulargewicht des T2-Proteins von 24 bis 36 KDa und ein Säure-pH-Optimum für die RNase-Aktivität gekennzeichnet ist.

2. Polynukleotid, das für eine rekombinante Ribonuklease der T2-Familie zur Verwendung bei der Hemmung der Tumor-Angiogenese in einer Testperson kodiert und in der Lage ist, diese in vivo zu exprimieren, wobei die Ribonuklease der T2-Familie Actin entweder in seiner aktiven oder nicht aktiven ribonukleolytischer Form bindet.

3. Ribonuklease nach Anspruch 1 oder Polynukleotid nach Anspruch 2, wobei die genannte Tumor-Angiogenese mit abnormalen Proliferationszellen assoziiert ist, die mit einer Proliferationserkrankung oder -krankheit assoziiert sind, ausgewählt aus der Gruppe bestehend aus Papillom, Blastogliom, Kaposi-Sarkom, Melanom, Lungenkrebs, Eierstockkrebs, Prostatakrebs, Plattenepithelkarzinom, Astrozytom, Kopfkrebs, Halskrebs, Blasenkrebs, Brustkrebs, Lungenkrebs, Kolorektalkrebs, Schilddrüsenkrebs, Bauchspeicheldrüsenkrebs, Magenkrebs, Hepatozellenkrebs, Leukämie, Lymphknotenerkrankung, Hodgkin-Lymphom, Burkittsche Erkrankung.

4. Ribonuklease nach Anspruch 1 oder Polynukleotid nach Anspruch 2 oder 3, wobei die genannte Ribonuklease der T2-Familie ausgewählt ist aus der Gruppe bestehend aus RNase HI0526, RNase I, RNase T2, RNase Rh, RNase M, RNase Trv, RNase Irp, RNase Irp1, RNase Le2, RNase Phyb, RNS2, RNase 3, RNase 1, RNase LE, RNase LX, S-RNase, RNase MC, RNase CL1, RNase BspI, RNase RCL2, RNase Dm, RNase Oy, RNase Tp und RNase 6P1.

5. Ribonuklease nach Anspruch 1 oder Polynukleotid nach Anspruch 2, wobei die genannte Tumor-Angiogenese mit einem bösartigen Tumor assoziiert ist.

6. Ribonuklease nach Anspruch 1 oder 5 oder Polynukleotid nach Anspruch 2, wobei die genannte Tumor-Angiogenese mit einem Primärtumor assoziiert ist.

7. Ribonuklease nach Anspruch 1 oder 6 oder Polynukleotid nach Anspruch 2, wobei die genannte Ribonuklease der T2-Familie eine *A niger* T2-RNase ist.

8. Ribonuklease nach Anspruch 1 oder 7 oder Polynukleotid nach Anspruch 2, wobei der genannten Ribonuklease der T2-Familie eine Ribonukleaseaktivität fehlt.

9. Ribonuklease nach Anspruch 1 oder 8 oder Polynukleotid nach Anspruch 2, wobei eine Actin-Bindungsaktivität des genannten Ribonukleaseproteins siedestabil ist.

10. Ribonuklease nach Anspruch 1 oder 9 oder Polynukleotid nach Anspruch 2, wobei die genannte Tumor-Angiogenese eine Angiogenese eines metastenbildenden Tumors ist.

11. Ribonuklease der T2-Familie zur Verwendung bei der Behandlung eines Zustands, der mit einer abnormalen Zellenproliferation in einer Testperson assoziiert ist, wobei die Ribonuklease der T2-Familie Actin entweder in seiner aktiven oder nicht aktiven ribonukleolytischen Form bindet, wobei die genannte Ribonuklease durch ein Molekulargewicht des T2-Proteins von 24 bis 36 KDa und ein Säure-pH-Optimum für die RNase-Aktivität gekennzeichnet ist und wobei der genannte Zustand ausgewählt ist aus der Gruppe bestehend aus Arthritis, Gelenkrheumatismus, Diabetes-Retinopathie, Restenose, in-Stent-Restenose und Arterienproteste-Restenose.

12. Polynukleotid, das für eine rekombinante Ribonuklease der T2-Familie zur Verwendung bei der Behandlung eines mit einer abnormalen Zellenproliferation in einer Testperson kodiert und in der Lage ist, diese in vivo zu exprimieren, wobei die Ribonuklease der T2-Familie Actin entweder in seiner aktiven oder nicht aktiven ribonukleolytischer Form bindet, wobei die genannte Ribonuklease durch ein Molekulargewicht des T2-Proteins von 24 bis 36 KDa und ein Säure-pH-Optimum für die RNase-Aktivität gekennzeichnet ist und wobei der genannte Zustand ausgewählt ist aus der Gruppe bestehend aus Arthritis, Gelenkrheumatismus, Diabetes-Retinopathie, Restenose, in-Stent-Restenose und Arterienproteste-Restenose.

## Revendications

1. Ribonucléase de la famille T2 destinée à être utilisée pour inhiber l'angiogenèse tumorale chez un sujet, où la ribonucléase de la famille T2 se lie à l'actine sous sa forme ribonucléolytique active ou non active, où ladite ribonucléase est **caractérisée par** un poids moléculaire de la protéine T2 de 24 à 36 kDa et par un pH acide optimal pour une activité RNase.

2. Polynucléotide codant pour et capable d'exprimer in vivo une ribonucléase recombinante de la famille T2 destinée à être utilisée pour inhiber l'angiogenèse tumorale chez un sujet, où la ribonucléase de la famille T2 se lie à l'actine sous sa forme ribonucléolytique active ou non active.

3. Ribonucléase selon la revendication 1 ou polynucléotide selon la revendication 2, où ladite angiogenèse tumorale est associée à des cellules proliférant de manière anormale associées à un trouble ou à une maladie proliférative sélectionné(e) dans le groupe consistant en un papillome, un glioblastome, le sarcome de Kaposi, un mélanome, le cancer du poumon, le cancer de l'ovaire, le cancer de la prostate, un carcinome à cellules squameuses, un astrocytome, le cancer de la tête, le cancer du cou, le cancer de la vessie, le cancer du sein, le cancer du poumon, le cancer colorectal, le cancer de la thyroïde, le cancer pancréatique, le cancer gastrique, un carcinome hépatocellulaire, une leucémie, un lymphome, la maladie de Hodgkin, la maladie de Burkitt.

4. Ribonucléase selon la revendication 1 ou polynucléotide selon la revendication 2 ou 3, où ladite ribonucléase de la famille T2 est sélectionnée dans le groupe consistant en la RNase HI0526, RNase I, RNase T2, RNase Rh, RNase M, RNAse Trv, RNAse Irp, RNAse Irp1, RNAse Le2, RNAse Phyb, RNS2, RNAse 3, RNAse 1, RNAse LE, RNAse LX, S-RNase, RNAse MC, RNAse CL1, RNAse Bsp1, RNAse RCL2, RNAse Dm, RNAse Oy, RNAse Tp et RNAse 6PL.

5. Ribonucléase selon la revendication 1 ou polynucléotide selon la revendication 2, où ladite angiogenèse tumorale est associée à une tumeur maligne.

6. Ribonucléase selon la revendication 1 ou 5 ou polynucléotide selon la revendication 2, où ladite angiogenèse tumorale est associée à une tumeur primaire.

7. Ribonucléase selon la revendication 1 ou 6 ou polynucléotide selon la revendication 2, où ladite ribonucléase de la famille T2 est la RNase T2 de *A. niger*.

8. Ribonucléase selon la revendication 1 ou 7 ou polynucléotide selon la revendication 2, où ladite ribonucléase de la famille T2 est dépourvue d'activité ribonucléase.

9. Ribonucléase selon la revendication 1 ou 8 ou polynucléotide selon la revendication 2, où l'activité de liaison à l'actine de ladite protéine ribonucléase est stable à l'ébullition.

10. Ribonucléase selon la revendication 1 ou 9 ou polynucléotide selon la revendication 2, où ladite angiogenèse tumorale est une angiogenèse d'une tumeur métastatique.

11. Ribonucléase de la famille T2 destinée à être utilisée pour traiter un état pathologique associé à une prolifération cellulaire anormale chez un sujet, où la ribonucléase de la famille T2 se lie à l'actine sous sa forme ribonucléolytique active ou non active, où ladite ribonucléase est **caractérisée par** un poids moléculaire de la protéine T2 de 24 à 36 kDa et par un pH acide optimal pour une activité RNase, et où ledit état pathologique est sélectionné dans le groupe consistant en l'arthrite, la polyarthrite rhumatoïde, la rétinopathie diabétique, une resténose, une resténose intra-stent et une resténose de greffe vasculaire.

12. Polynucléotide codant pour et capable d'exprimer in vivo une ribonucléase recombinante de la famille T2 destinée à être utilisée pour traiter un état pathologique associé à une prolifération cellulaire anormale chez un sujet, où la ribonucléase de la famille T2 se lie à l'actine sous sa forme ribonucléolytique active ou non active, où ladite ribonucléase est **caractérisée par** un poids moléculaire de la protéine T2 de 24 à 36 kDa et par un pH acide optimal pour une activité RNase, et où ledit état pathologique est sélectionné dans le groupe consistant en l'arthrite, la polyarthrite rhumatoïde, la rétinopathie diabétique, une resténose, une resténose intra-stent et une resténose de greffe vasculaire.
